# EUROPEAN PATENT APPLICATION

(11) **EP 4 759 245 A1**
(43) Date of publication of application: **17.06.2026**
(21) Application number: 24871698.7
(22) Date of filing: 29.08.2024
(51) Int. Cl.: A61B 17/135

(54) **HEMOSTATIC INSTRUMENT AND HEMOSTATIC INSTRUMENT SET**

(30) Priority: 29.09.2023 JP 2023170329
(71) Applicant: Terumo Kabushiki Kaisha, Tokyo 151-0072 (JP)
(72) Inventor: HAYASHI, Koki, Fujinomiya-shi, Shizuoka 418-0015 (JP); TANAKA, Akira, Fujinomiya-shi, Shizuoka 418-0015 (JP); KAWASUMI, Kenta, Fujinomiya-shi, Shizuoka 418-0015 (JP)
(74) Representative: Casalonga
(86) International application number: PCT/JP2024/030860
(87) International publication number: WO 2025/069897

(57) **Abstract**

To provide a hemostatic device and a hemostatic device set capable of preventing deterioration in sealing performance of a connector portion due to a foreign matter that has entered an inner cavity of the connector portion.

An injection member 200 of a hemostatic device 10 includes a connector portion 210 and a coupling portion 220 that couples an inner cavity of the connector portion and an inner cavity 170a of an inflatable member 170. A check valve member 300 included in the connector portion 210 includes an outer member 310, an inner member 320 located in an inner cavity 316 of the outer member, and a spring member 390 connected to the inner member. The inner member includes a main body 330 in which the spring member is in contact with an inner protrusion portion 315 in a natural state, and a distal protrusion portion 340 protruding from a distal end of the main body toward a distal opening 311 side. An upper end portion 350 included in the distal protrusion portion includes a recess portion 353 recessed toward the main body. A side wall portion 360 included in the distal protrusion portion includes a slit portion 361 continuous with the recess portion and extending in a longitudinal direction of the inner member, and the recess portion includes a trap portion 355 having a width W1 that narrows from a center position O1 of the upper end portion toward an outer edge 351 of the upper end portion in a plan view.

## Description

### Technical Field

The present invention relates to a hemostatic device and a hemostatic device set.

### Background Art

As one of catheter procedures, there is known a procedure in which various medical elongated bodies are introduced into a blood vessel of a limb such as an arm or hand of a patient through a puncture site formed by puncturing the blood vessel to perform a diagnosis or a therapy at a lesion site. For example, Patent Literature 1 discloses a hemostatic device for performing hemostasis at a puncture site formed to enable access to a blood vessel (including a distal radial artery) running in a hand.

A hemostatic device of Patent Literature 1 includes: an inflatable member that applies a compressive force to a puncture site formed on a hand of a patient; a cover member for securing the inflatable member to the hand of the patient; a connector portion (port portion) configured to be capable of injecting a fluid for inflating the inflatable member; and a coupling portion (tube portion) that connects the connector portion and an inner cavity of the inflatable member.

### Citation List

### Patent Literature

Patent Literature 1: US 2019/0133602 A

### Summary of Invention

### Technical Problem

When hemostasis is performed at a puncture site formed on a hand of the patient using the hemostatic device of Patent Literature 1, an operator such as a doctor (hereinafter, referred to as "operator") secures the hemostatic device to the hand of the patient in a state where the inflatable member is disposed at the puncture site formed on the hand of the patient. By inflating the inflatable member in a state where the hemostatic device is secured to the hand of the patient, the operator can perform hemostasis by applying a compressive force from the inflatable member to the puncture site formed on the hand of the patient.

When inflating the inflatable member, the operator connects, to the connector portion, a fluid injection device such as a syringe for injecting a fluid. Inside the connector portion of the hemostatic device, for example, a check valve (valve body) is disposed for blocking fluid communication between the inner cavity of the inflatable member and the outside of the connector portion in a state where the fluid injection device is not connected. The operator connects the fluid injection device to the connector portion and pushes the check valve disposed inside the connector portion with the fluid injection device, thereby moving the check valve from a predetermined closed position to a predetermined open position of the connector portion. By moving the check valve to the predetermined open position, the operator can cause the fluid holding portion (external tubular portion) of the fluid injection device and the inner cavity of the inflatable member to be in fluid communication with each other via a connection portion (distal tubular portion) of the fluid injection device. The operator can inflate the inflatable member by operating the fluid injection device to inject the fluid into the inner cavity of the inflatable member in a state where the fluid holding portion of the fluid injection device and the inner cavity of the inflatable member are in fluid communication with each other.

After inflating the inflatable member, the operator withdraws the fluid injection device from the connector portion. When the operator withdraws the fluid injection device from the connector portion, the check valve disposed inside the connector portion moves to the closed position in the connector portion. The hemostatic device described above blocks fluid communication between the inner cavity of the inflatable member and the outside of the connector portion again when the check valve is moved to the closed position in the connector portion.

In the connector portion included in the hemostatic device of Patent Literature 1, it is possible to adopt a configuration in which a part of the fluid injection device can be inserted into the connector portion as described above. Furthermore, in a case where the connector portion is adopted, the hemostatic device can be provided with a connection opening for maintaining a connection state between the connector portion and the fluid injection device. In a case where the structure of the connector portion and the connection opening is adopted in the hemostatic device, in a state where the fluid injection device is not connected to the connector portion, a part of the inside of the connector portion is exposed to the outside of the connector portion through the connection opening. As described above, when a part of the inside of the connector portion is exposed to the outside of the connector portion through the connection opening, foreign matter may enter the inside of the connector portion through the connection opening. Therefore, in a case where the structure of the connector portion and the connection opening described above is adopted in the hemostatic device, when an operation to move the check valve from the open position to the closed position is performed in a state where the foreign matter enters the inside of the connector portion, there is a possibility that the foreign matter is caught between the check valve moved to the closed position and the inner wall of the connector portion. Therefore, in the above-described hemostatic device, in a case where the structure of the connector portion and the connection opening is adopted, when the foreign matter is caught between the check valve moved to the closed position and the inner wall of the connector portion, the sealing performance (airtightness) by the check valve is degraded, and there is a possibility that the fluid injected into the inner cavity of the inflatable member leaks to the outside via the check valve and the connector portion. In the hemostatic device described above, when the fluid injected into the inner cavity of the inflatable member leaks to the outside of the connector portion as described above, the compressive force applied by the inflatable member to the puncture site formed in the hand of the patient is reduced. Therefore, the hemostatic device of Patent Literature 1 may not be capable of exhibiting a sufficient hemostatic effect due to the influence of the foreign matter that has entered the inside of the connector portion.

The present invention has been made in view of the above problems, and an object of the present invention is to provide a hemostatic device and a hemostatic device set capable of preventing degradation in sealing performance of a connector portion due to foreign matter that has entered the inner cavity of the connector portion.

### Solution to Problem

The present invention is achieved by any one of the following aspects (1) to (13).
(1) A hemostatic device including: a cover member configured to cover a puncture site formed on a patient; an inflatable member connected to the cover member and configured to compress the puncture site; and an injection member connected to the inflatable member and configured to be capable of injecting a fluid into the inflatable member, in which the injection member includes a connector portion, and a coupling portion that couples an inner cavity of the connector portion and an inner cavity of the inflatable member, the connector portion includes a check valve member configured to be capable of controlling communication between the inner cavity of the connector portion and an outside, and a connection member that connects the check valve member and the coupling portion, the check valve member includes an outer member, an inner member located in an inner cavity of the outer member, and a spring member connected to the inner member, the outer member includes a distal opening configured to expose a part of the inner member to the outside, a proximal opening communicating between the inner cavity of the outer member and an inner cavity of the connection member, and an inner protrusion portion configured to prevent movement of the inner member toward the distal opening side, the spring member is located between the proximal opening and the inner protrusion portion so as to maintain contact between an outer surface of the inner member and the inner protrusion portion in a natural state, the inner member includes a main body configured to contact the inner protrusion portion in the natural state, and a distal protrusion portion protruding from a distal end of the main body toward the distal opening side, the distal protrusion portion includes an upper end portion and a side wall portion connecting the upper end portion and the main body, the upper end portion includes a recess portion recessed toward the main body side, the side wall portion includes a slit portion continuous with the recess portion and extending in a longitudinal direction of the inner member, and the recess portion includes a trap portion having a width that narrows from a center position of the upper end portion toward an outer edge of the upper end portion in a plan view.
(2) The hemostatic device according to (1), in which the recess portion is connected to the slit portion via the trap portion.
(3) The hemostatic device according to (1) or (2), in which the upper end portion includes a first projection formed at the outer edge of the upper end portion.
(4) The hemostatic device according to (3), in which the upper end portion includes a plurality of upper surface portions located on a same plane.
(5) The hemostatic device according to (4), in which the upper surface portion includes a second projection formed at an inner edge of the upper surface portion.
(6) The hemostatic device according to any one of (1) to (5), in which an outer diameter of the upper end portion is greater than an inner diameter of the distal opening.
(7) The hemostatic device according to any one of (1) to (6), in which the spring member holds an end of the slit portion located on the main body side at a position on the distal opening side relative to the inner protrusion portion in the natural state, and the spring member moves the end of the slit portion to a position on the proximal opening side relative to the inner protrusion portion when a pushing force in a direction toward the proximal opening is applied to the spring member.
(8) The hemostatic device according to any one of (1) to (7), in which the recess portion includes a cutout portion connecting the center position of the upper end portion and a side surface of the upper end portion, and the cutout portion has a width that gradually narrows toward the main body side.
(9) The hemostatic device according to (8), in which the cutout portions are disposed at four locations at equal intervals in a circumferential direction of the upper end portion with reference to the center position of the upper end portion.
(10) The hemostatic device according to any one of (1) to (9), in which the slit portion has a predetermined depth along a direction from the side wall portion side toward the center position side of the upper end portion.
(11) The hemostatic device according to any one of (1) to (10), in which the slit portion extends with a substantially constant width along a longitudinal direction of the side wall portion.
(12) The hemostatic device according to any one of (1) to (11), in which the upper end portion includes a first region including the upper surface portion and a second region forming a bottom portion of the recess portion, and an end of the slit portion located on the main body side is located on the main body side relative to the second region.
(13) A hemostatic device set including: the hemostatic device according to any one of (1) to (12); and a fluid injection device including a distal tubular portion that abuts the upper end portion, in which an upper surface portion that the distal tubular portion abuts in the upper end portion has an outer diameter greater than that of the distal tubular portion in a plan view.

### Advantageous Effects of Invention

The hemostatic device according to (1) includes the check valve member disposed in the inner cavity of the connector portion. In the natural state, the spring member of the check valve member forms the seal portion inside the connector portion by maintaining contact between the inner member and the inner protrusion portion formed in the outer member, and prevents the inner cavity of the inflatable member from being in fluid communication with the outside of the connector portion via the connector portion. Furthermore, in the hemostatic device, the inner member includes the main body configured to be in contact with the inner protrusion portion of the outer member, and the distal protrusion portion that protrudes from the distal end of the main body toward the distal opening side. The distal protrusion portion includes the upper end portion and the side wall portion connecting the upper end portion and the main body. The upper end portion has the recess portion recessed toward the main body side, and the recess portion has the trap portion having the width that narrows from the center position of the upper end portion toward the outer edge of the upper end portion in a plan view. In the hemostatic device described above, the foreign matter that has entered the inner cavity of the outer member through the distal opening can be captured by the trap portion included in the recess portion located on the distal opening side relative to the main body of the inner member. Furthermore, in the hemostatic device, when the fluid is fed to the injection member via the connector portion in a state where the foreign matter has entered the connector portion, the foreign matter moved to the main body side by the injected fluid can be captured by the trap portion. Therefore, the hemostatic device can suppress movement of the foreign matter having entered the inner cavity of the outer member to the main body side of the inner member. Thus, even in a case where the foreign matter enters the inner cavity of the outer member, the hemostatic device can prevent the foreign matter from being caught between the outer surface of the main body of the inner member and the inner protrusion portion of the outer member when the spring member is in the natural state. Therefore, the hemostatic device can prevent the sealing performance of the seal portion from degrading due to the influence of the foreign matter that has entered the inner cavity of the outer member. Thus, the hemostatic device can prevent a decrease in the compressive force applied by the inflatable member to the puncture site formed in the patient due to the influence of the foreign matter that has entered the inner cavity of the outer member. Furthermore, in the hemostatic device, the side wall portion included in the distal protrusion portion has the slit portion which is continuous with the recess portion and extends in the longitudinal direction of the inner member. In the hemostatic device, when a pushing force in a direction away from the distal opening is applied to the spring member via the fluid injection device inserted into the inner cavity of the outer member at the time of injecting the fluid into the inner cavity of the inflatable member, the spring member is compressed along the longitudinal direction of the connector portion. When the spring member is compressed, the hemostatic device moves the inner member to the proximal side in accordance with the compression of the spring member. When the inner member moves toward the proximal side in accordance with the compression of the spring member, the contact between the outer surface of the main body of the inner member and the inner protrusion portion of the outer member is released. In the hemostatic device, when the fluid is injected into the inner cavity of the outer member via the fluid injection device in a state where the contact between the outer surface of the main body of the inner member and the inner protrusion portion of the outer member is released, the injected fluid can be smoothly moved from the distal protrusion portion side toward the main body side via the slit portion formed in the side wall portion connecting the upper end portion and the main body. Therefore, when the inflatable member is inflated using the fluid injection device, the hemostatic device can smoothly feed the fluid into the inner cavity of the inflatable member via the connector portion and the coupling portion.

### Brief Description of Drawings

[Fig. 1] Fig. 1 is a plan view illustrating a hemostatic device according to an embodiment as viewed from an outer surface side of each band body.
[Fig. 2] Fig. 2 is a plan view illustrating a hemostatic device according to the embodiment as viewed from an inner surface side of each band body.
[Fig. 3] Fig. 3 is an enlarged view illustrating a part of a hemostatic device as viewed from an outer surface side of a main body of a cover member.
[Fig. 4] Fig. 4 is an enlarged view illustrating a part of a hemostatic device as viewed from an inner surface side of a main body of a cover member.
[Fig. 5] Fig. 5 is an enlarged view illustrating a part of a hemostatic device as viewed from an outer surface side of a main body of a cover member.
[Fig. 6] Fig. 6 is a partial cross-sectional view of a hemostatic device taken along arrow 6A-6A illustrated in Fig. 5, and is a view illustrating a state when an inflatable member inflates.
[Fig. 7] Fig. 7 is a partial cross-sectional view of a hemostatic device taken along arrow 7A-7A illustrated in Fig. 5, and is a view illustrating a state when an inflatable member inflates.
[Fig. 8] Fig. 8 is an enlarged perspective view illustrating a part of an injection member.
[Fig. 9] Fig. 9 is a cross-sectional view of a check valve member taken along arrow 9A-9A illustrated in Fig. 8.
[Fig. 10] Fig. 10 is a cross-sectional view of a check valve member.
[Fig. 11] Fig. 11 is a cross-sectional view of a check valve member.
[Fig. 12] Fig. 12 is a partial cross-sectional view of an inner member, which is an enlarged view of a portion 12A illustrated in Fig. 11.
[Fig. 13] Fig. 13 is a perspective view of an inner member.
[Fig. 14] Fig. 14 is a partial cross-sectional view of a distal protrusion portion of an inner member.
[Fig. 15] Fig. 15 is a plan view of an inner member as viewed from a distal side.
[Fig. 16] Fig. 16 is a view illustrating a side wall portion of an inner member.
[Fig. 17] Fig. 17 is a plan view of a connector portion as viewed from a distal side.
[Fig. 18] Fig. 18 is a perspective view illustrating a state when a distal tubular portion of a fluid injection device is pressed against an upper end portion of an inner member.
[Fig. 19] Fig. 19 is a view illustrating a hand (right hand) of a patient as a use target of a hemostatic device.
[Fig. 20] Fig. 20 is a view schematically illustrating a usage example of a hemostatic device.
[Fig. 21] Fig. 21 is a view schematically illustrating a usage example of a hemostatic device.
[Fig. 22] Fig. 22 is a view schematically illustrating a usage example of a hemostatic device.
[Fig. 23] Fig. 23 is a partial cross-sectional view taken along arrow 23A-23A illustrated in Fig. 22.
[Fig. 24] Fig. 24 is a partial cross-sectional view taken along arrow 24A-24A illustrated in Fig. 22.
[Fig. 25] Fig. 25 is a view illustrating a side wall portion of an inner member according to a first modification example.
[Fig. 26] Fig. 26 is a plan view of an inner member according to a second modification example as viewed from a distal side.
[Fig. 27] Fig. 27 is a side view of an inner member according to the second modification example.
[Fig. 28] Fig. 28 is a perspective view illustrating a distal protrusion portion of an inner member according to the second modification example.
[Fig. 29] Fig. 29 is a partial cross-sectional view of a distal protrusion portion of an inner member according to the second modification example.
[Fig. 30] Fig. 30 is a perspective view illustrating a distal protrusion portion of an inner member according to a third modification example.
[Fig. 31] Fig. 31 is a plan view of an inner member according to a fourth modification example as viewed from a distal side.
[Fig. 32] Fig. 32 is a side view of an inner member according to the fourth modification example.
[Fig. 33] Fig. 33 is a perspective view illustrating a distal protrusion portion of an inner member according to the fourth modification example.
[Fig. 34] Fig. 34 is a partial cross-sectional view of a distal protrusion portion of an inner member according to the fourth modification example.
[Fig. 35] Fig. 35 is a plan view of an inner member according to a fifth modification example as viewed from a distal side.
[Fig. 36] Fig. 36 is a side view of an inner member according to the fifth modification example.
[Fig. 37] Fig. 37 is a perspective view illustrating a distal protrusion portion of an inner member according to the fifth modification example.
[Fig. 38] Fig. 38 is a partial cross-sectional view of a distal protrusion portion of an inner member according to the fifth modification example.

### Description of Embodiments

An embodiment of the present invention will be described below with reference to the accompanying drawings. The following description does not limit the technical scope or the significance of each term set forth in the claims. Furthermore, the dimensional ratios in the drawings are exaggerated for convenience of description and may be different from actual ratios.

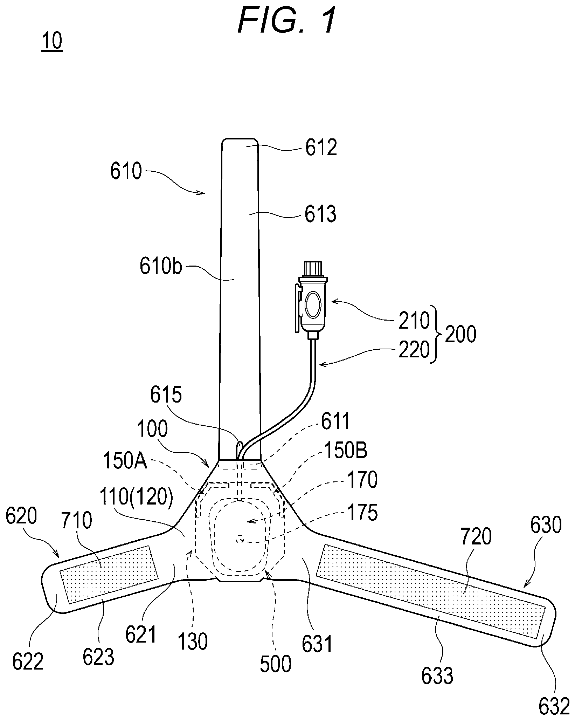

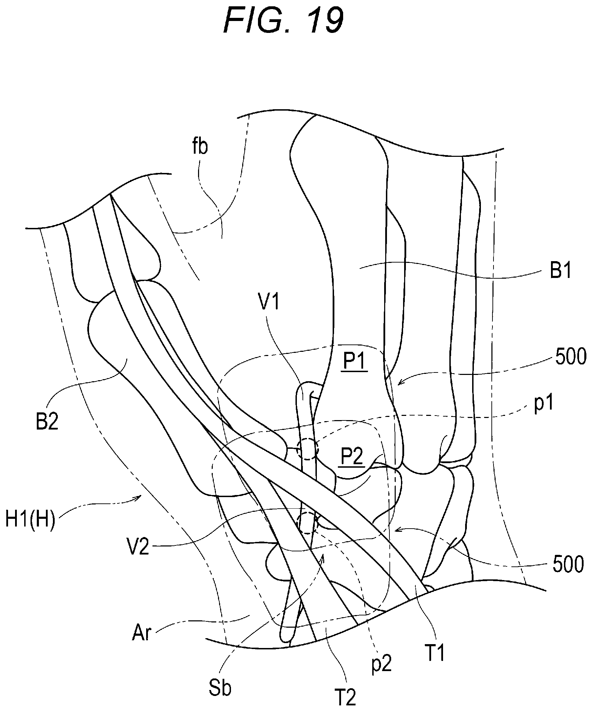

Figs. 1 to 18 are views illustrating a hemostatic device 10 according to an embodiment. Figs. 19 to 24 are views illustrating usage examples of the hemostatic device 10 according to the embodiment.

For example, as illustrated in Figs. 19, 22, 23, and 24, the hemostatic device 10 can be used to perform hemostasis at a first puncture site p1 formed in a hand H located on a distal side (fingertip side) with respect to a forearm Ar of a patient when a sheath tube 810 of an introducer 800 placed at the first puncture site p1 (hereinafter, also referred to as a "first puncture site") is withdrawn.

The specific position of the puncture site, which is a hemostasis target of the hemostatic device 10 is not particularly limited, but in the present specification, the first puncture site p1 and a second puncture site p2 are described as an example.

As illustrated in Figs. 19, 22, 23, and 24, the first puncture site p1 is a puncture site formed in a distal radial artery (hereinafter, also referred to as a "blood vessel V1") located on a distal side of a snuff box Sb of a palmar artery running on a dorsal side of a right hand H1 located on the distal side of the forearm Ar of the patient. Furthermore, the first puncture site p1 is located at a predetermined position, which is a position between a metacarpal bone B1 of an index finger and a metacarpal bone B2 of a thumb and avoiding the metacarpal bone B1 of the index finger and the metacarpal bone B2 of the thumb. Note that the snuff box Sb is a cavity in the hand located in the vicinity of the radius when the patient extends the thumb of the hand H.

As illustrated in Fig. 19, the second puncture site p2 is a puncture site formed in an artery V2 located in the snuff box Sb of the palmar artery running on the dorsal side of the right hand H1. The second puncture site p2 is located on the forearm Ar side (proximal side) of the patient with respect to the first puncture site p1, and is located between an extensor pollicis longus muscle tendon T1 and an extensor pollicis brevis muscle tendon T2 located on the dorsal side of the right hand H1 of the patient. Furthermore, the second puncture site p2 is located at a predetermined position avoiding the position of the metacarpal bone B1 of the index finger and the position of the metacarpal bone B2 of the thumb.

Note that the hemostatic device 10 can also be applied to hemostasis of a puncture site on the left hand of the patient formed at a position corresponding to the first puncture site p1 on the right hand H1 of the patient or a puncture site on the left hand of the patient formed at a position corresponding to the second puncture site p2 on the right hand H1 of the patient.

Hereinafter, the hemostatic device 10 will be described in detail.

### (Hemostatic Device 10)

As illustrated in Figs. 1, 2, 8, 22, 23, and 24, the hemostatic device 10 generally includes a cover member 100 configured to cover the first puncture site p1, an inflatable member 170 connected to the cover member 100 and configured to compress the first puncture site p1, an injection member 200 connected to the inflatable member 170 and configured to be capable of injecting a fluid into the inflatable member 170.

In the present specification, each direction regarding the cover member 100 and the support member 500 of the hemostatic device 10 is defined as follows. In the present specification, the fingertip side of a finger is referred to as a "distal side", and the forearm Ar side is referred to as a "proximal side" in a state where the hemostatic device 10 is worn on the right hand H1 of the patient.

### (Inflatable Member 170)

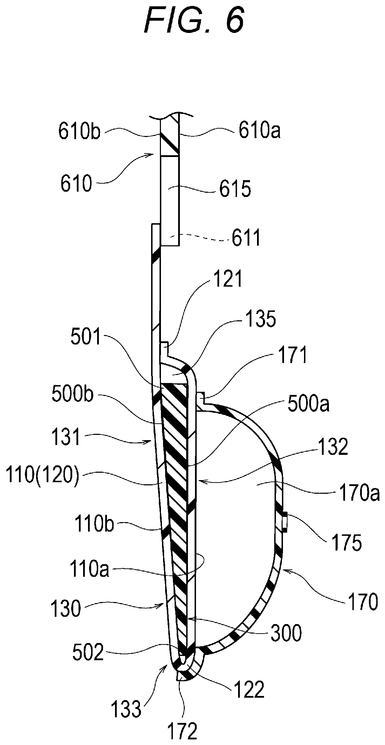

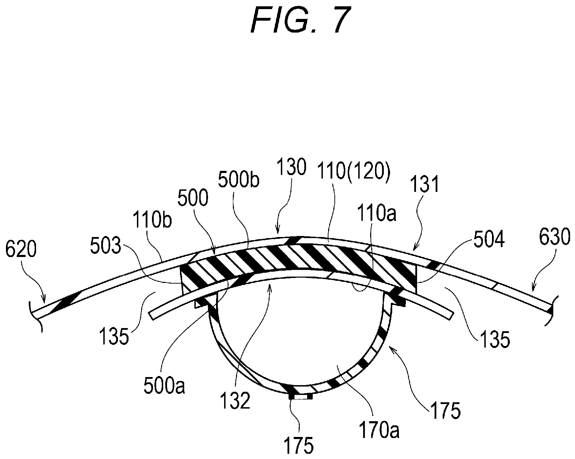

As illustrated in Figs. 6 and 7, the inflatable member 170 includes an inner cavity 170a into which the fluid can be injected. The inflatable member 170 is configured to be inflated as the fluid is injected into the inner cavity 170a and deflated as the fluid injected into the inner cavity 170a is discharged.

The fluid used for the inflation of the inflatable member 170 is, for example, a gas such as air. Figs. 6 and 7 illustrate cross-sectional views of the inflatable member 170 in an inflated state.

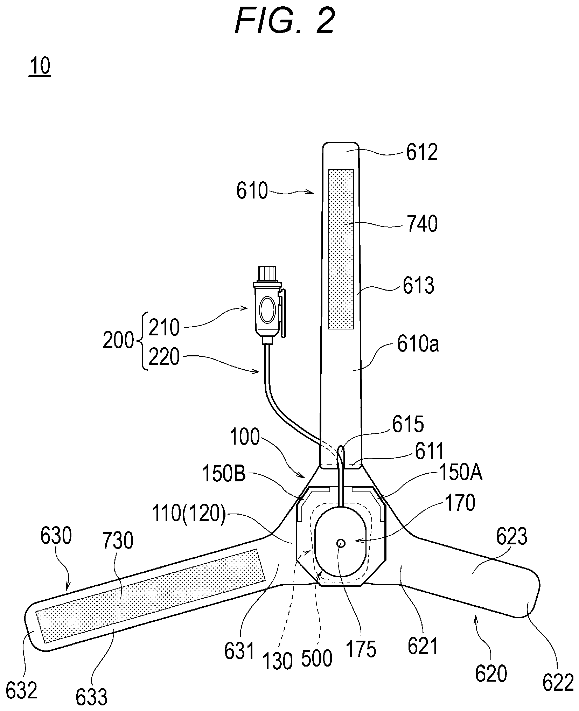

A coupling portion 220 (see Figs. 1 and 2) to be described later is coupled to the inner cavity 170a of the inflatable member 170. Note that, in the cross-sectional views of Figs. 6, 7, 23, and 24, the coupling portion 220 is not illustrated.

As illustrated in Figs. 6 and 7, the inflatable member 170 can include, for example, a film-shaped member (sheet material) connected to a sheet material 120 that constitutes a cover main body 110 of the cover member 100.

The sheet material constituting the inflatable member 170 can be connected to the cover member 100 such that the inner cavity 170a into which the fluid can be injected is defined with a lower surface region 132 of a holding portion 130 made of the sheet material 120 constituting the cover main body 110 of the cover member 100.

An outer peripheral edge of the film-shaped member forming the inflatable member 170 defines an edge of the inflatable member 170. The edge of the inflatable member 170 can be connected to the sheet material 120 by, for example, fusing, using an adhesive, or the like.

As illustrated in Fig. 6, an edge 172 located at a lower end portion 502 of the support member 500 in the sheet material constituting the inflatable member 170 can be connected to a curved region 133 formed by the cover member 100. Furthermore, as illustrated in Fig. 6, an edge 171 located on an upper end portion 501 side of the support member 500 in the sheet material constituting the inflatable member 170 can be secured to the sheet material 120 at a predetermined position overlapping a lower surface 500a of the support member 500 in the plane direction.

The film-shaped member constituting the inflatable member 170 can be made of, for example, a resin material having a predetermined thickness.

A material for the film-shaped member constituting the inflatable member 170 is not particularly limited, and examples thereof include polyvinyl chloride, ethylene-vinyl acetate copolymer (EVA), polyvinylidene chloride, silicone, polyamide (nylon), polyurethane, polyolefin such as polyethylene, polypropylene, or polybutadiene, polyester such as polyethylene terephthalate (PET) or polybutylene terephthalate (PBT), various thermoplastic elastomers such as polyamide elastomer (nylon elastomer), polyurethane elastomer, and polyester elastomer, or any combination thereof (for example, blended resin, polymer alloy, laminate, or the like).

As illustrated in Figs. 6 and 7, the inflatable member 170 is disposed on an inner surface 110a side of the cover main body 110 of the cover member 100.

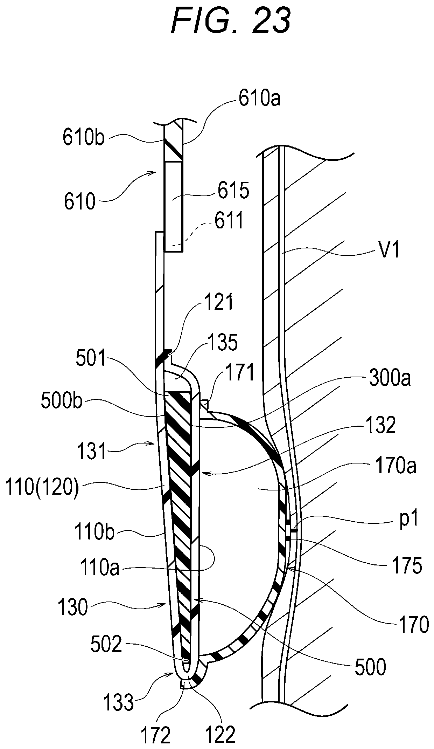

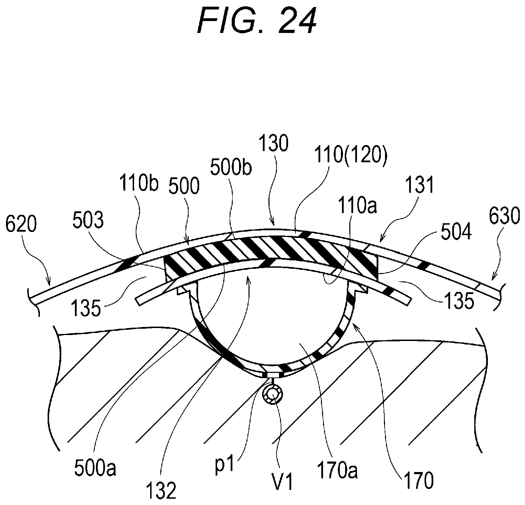

In the present embodiment, the inner surface 110a of the cover main body 110 is defined by a surface to be disposed on a body surface side of the right hand H1 in a lower surface region 132 of the holding portion 130 (see Figs. 23 and 24). Furthermore, an outer surface 110b of the cover main body 110 is defined by a surface on a side facing the outside in an upper surface region 131 of the holding portion 130.

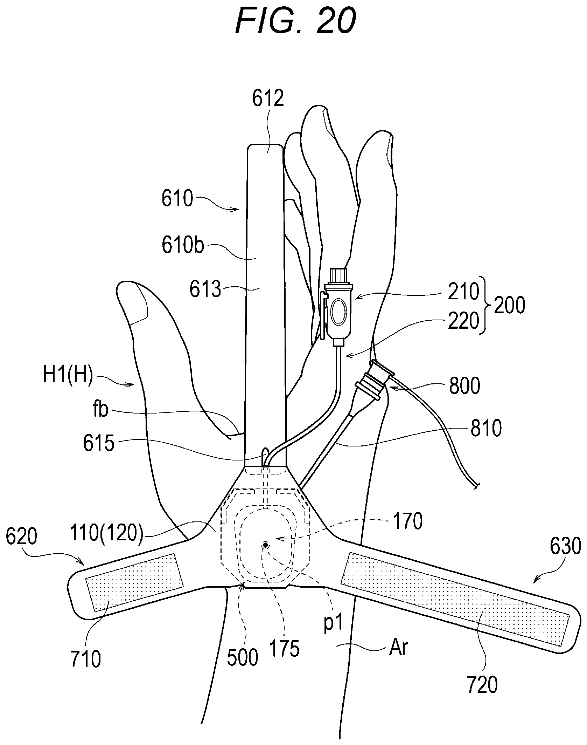

The inflatable member 170 includes a marker portion 175 for aligning the inflatable member 170 with the first puncture site p1 as illustrated in Figs. 20, 21, and 22.

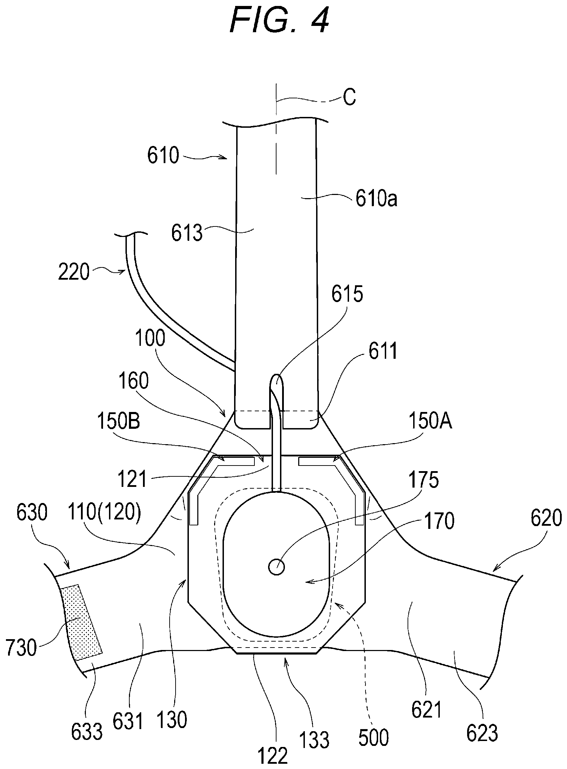

As illustrated in Figs. 4, 6, and 7, the marker portion 175 can include a transparent central portion surrounding the center position of the inflatable member 170 and a colored circular frame portion surrounding the central portion.

The marker portion 175 is disposed on an outer surface of the inflatable member 170 as illustrated in Figs. 6 and 7. Note that the marker portion 175 may be disposed on an inner surface of the inflatable member 170 facing the inner cavity 170a.

The specific shape and color of the marker portion 175, a position of the marker portion 175 in the plane direction of the inflatable member 170, a method for forming the marker portion 175, and the like are not particularly limited. The marker portion 175 can also be constituted by, for example, a circular marker that is entirely colored, a marker including a transparent central portion and a rectangular frame portion, a rectangular marker that is entirely colored, or the like.

However, the planar shape of the inflatable member 170 in plan views illustrated in Figs. 6 and 7 is not particularly limited. The inflatable member 170 can be configured to have, for example, a planar shape such as a circular shape, an elliptical shape, or a rectangular shape. In a case where the inflatable member 170 is configured to have a planar shape such as a circular shape, an elliptical shape, or a rectangular shape, the center of the inflatable member 170 can be defined by a center position of the outer shape in a plan view.

Furthermore, the specific configuration of the inflatable member 170 is not particularly limited. For example, the inflatable member 170 may be formed by joining an edge of one of the sheet-like members to an edge of the other of the sheet-like members in a state where the inner cavity 170a is formed between two sheet-like members. In a case where the inflatable member 170 is constituted as described above, the surface of the one of the sheet-like members of the inflatable member 170 can be connected to the sheet material 120 constituting the cover main body 110 of the cover member 100. Furthermore, the inflatable member 170 can also be constituted by, for example, one bag-shaped member shaped to include the inner cavity 170a inside. In a case where the inflatable member 170 is constituted as described above, a part of the surface of the bag-shaped member of the inflatable member 170 can be connected to the sheet material 120 constituting the cover main body 110 of the cover member 100.

### (Cover Member 100)

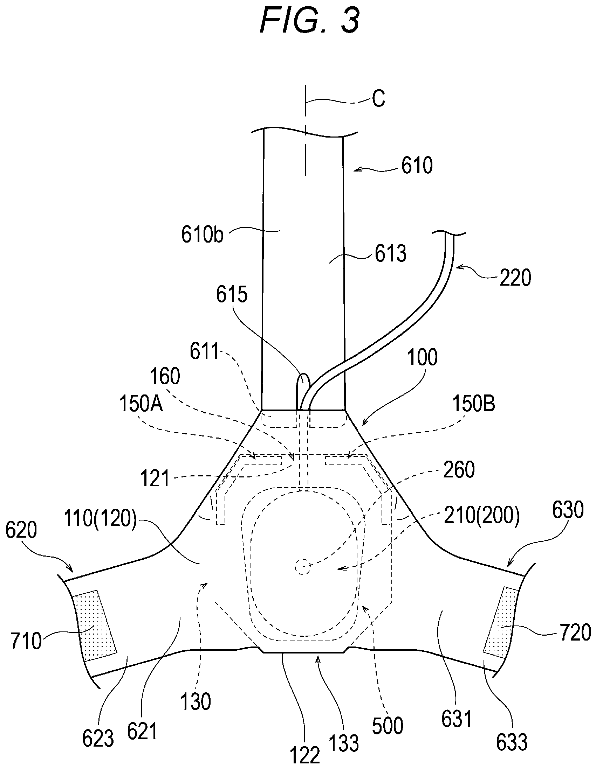

As illustrated in Figs. 3, 4, 5, and 22, the cover member 100 includes the cover main body 110 in which the inflatable member 170 is located, a first band body 610 that is configured to be disposed between fingers of the patient and extends from the cover main body 110 in a first direction, a second band body 620 extending from the cover main body 110 in a second direction different from the first direction, and a third band body 630 facing the second band body 620 across the inflatable member 170 and extending from the cover main body 110 in a third direction different from the first direction and the second direction.

A region of the cover member 100 where the inflatable member 170 is disposed (a region overlapping the inflatable member 170 in the plan views illustrated in Figs. 3, 4, and 5) defines the cover main body 110.

As illustrated in Figs. 6 and 7, the cover main body 110 has a two-layer structure. The cover main body 110 having a two-layer structure includes the holding portion 130 that holds the support member 500.

As illustrated in Figs. 6 and 7, the holding portion 130 includes the upper surface region 131 located on the upper surface 500b side of the support member 500, the lower surface region 132 that is located on the lower surface 500a side of the support member 500 and faces the upper surface region 131 across the support member 500, and the curved region 133 connecting the upper surface region 131 and the lower surface region 132 on the proximal side of the support member 500 (the lower end portion 502 side of the support member 500).

The curved region 133 is formed by folding back a part of the sheet material 120 constituting the cover main body 110 toward the first band body 610. In the present embodiment, a part of the sheet material 120 constituting the cover main body 110 is folded back from the proximal side to the distal side of the cover main body 110. An insertion portion 135 in which the support member 500 can be disposed is formed between the upper surface region 131 and the lower surface region 132 of the sheet material 120 folded back in this manner. A portion of the sheet material 120 where the insertion portion 135 is formed constitutes the holding portion 130 for holding the support member 500.

As illustrated in Fig. 6, the edge 121 (hereinafter, referred to as "edge 121 of the lower surface region 132") located on the distal side of the folded portion of the sheet material 120 constituting the cover main body 110 is fixed to the sheet material 120 by means of secured regions 150A and 150B to be described later at a position on the distal side with respect to the upper end portion 501 of the support member 500.

The proximal portion 122 of the cover member 100, which is formed of a part of the sheet material 120 constituting the cover main body 110, is located in the curved region 133.

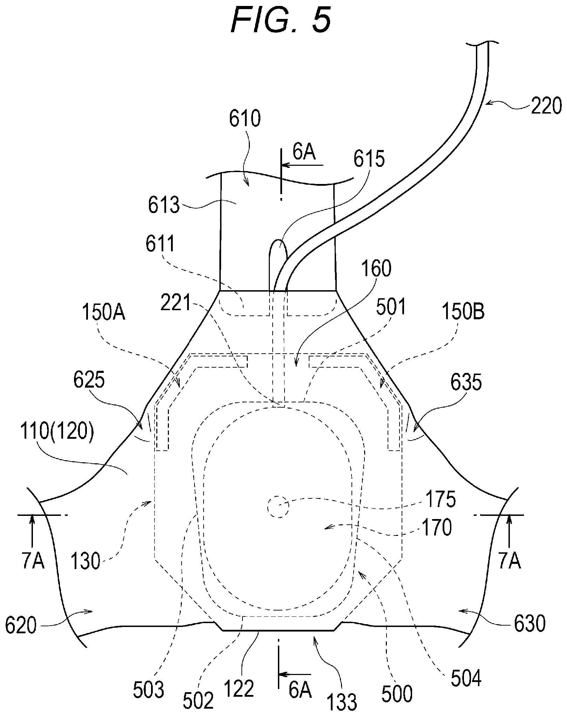

As illustrated in Figs. 5 and 6, the insertion portion 135 of the holding portion 130 is closed at a position on the upper end portion 501 side of the support member 500 except for a non-secured region 160 to be described later.

Furthermore, as illustrated in Figs. 5 and 6, a portion of the insertion portion 135 of the holding portion 130 on the lower end portion 502 side of the support member 500 is closed by the curved region 133.

As illustrated in Figs. 4 and 5, the edge 121 of the lower surface region 132 includes a first secured region 150A and a second secured region 150B, which are secured to the upper surface region 131, and a non-secured region 160 adjacent to each of the secured regions 150A and 150B and not secured to the upper surface region 131.

For example, the first secured region 150A and the second secured region 150B can be disposed to face each other across the non-secured region 160 in the plan views illustrated in Figs. 4 and 5.

The first secured region 150A and the second secured region 150B can be configured to have bilaterally symmetrical shapes with respect to a center line C (see Fig. 4) of the first band body 610. The secured regions 150A and 150B can be constituted by, for example, an adhesive portion or a fused portion that secures the lower surface region 132 to the sheet material 120.

Each of the secured regions 150A and 150B can be disposed such that at least a part of each of the secured regions 150A and 150B is located on the distal side of the support member 500. Furthermore, each of the secured regions 150A and 150B can be disposed so as to surround each edge portion on the distal side of the support member 500 in plan views illustrated in Figs. 4 and 5.

Each of the secured regions 150A and 150B can be disposed such that at least a part of each of the secured regions 150A and 150B extends from the upper end portion 501 of the support member 500 to the side surface portions 503 and 504 of the support member 500.

Note that the specific shape, position, and the like of each of the secured regions 150A and 150B are not particularly limited, and can be arbitrarily changed.

### (First Band Body 610, Second Band Body 620, Third Band Body 630)

The first band body 610 is connected to the cover main body 110 at a position facing the curved region 133 across the support member 500 as illustrated in Figs. 3 and 4.

The second band body 620 and the third band body 630 are integrally formed with the cover main body 110.

Note that similar to the first band body 610, the second band body 620 and the third band body 630 may be formed of a member different from the cover main body 110 and connected to the cover main body 110. Furthermore, the first band body 610 may be formed integrally with the cover main body 110 similarly to the second band body 620 and the third band body 630.

As illustrated in Figs. 1, 2, 3, and 4, the first band body 610 includes a first end 611 connected to the cover main body 110 of the cover member 100, a second end 612 located opposite to the first end 611, and an intermediate portion 613 extending between the first end 611 and the second end 612.

As illustrated in Fig. 6, the first end 611 of the first band body 610 is connected to the cover main body 110 of the cover member 100. The first end 611 of the first band body 610 can be connected to the cover main body 110 by, for example, fusion or adhesion.

As illustrated in Figs. 3 and 4, the first band body 610 has a slit 615 extending in the longitudinal direction of the first band body 610.

As illustrated in Fig. 6, the slit 615 penetrates an inner surface 610a and an outer surface 610b of the first band body 610.

As illustrated in Figs. 3 and 4, the coupling portion 220 of the injection member 200 passes between the secured regions 150A and 150B, which are formed in the cover main body 110, and is inserted from the inner surface 610a side of the first band body 610 to the outer surface 610b side of the first band body 610 via the slit 615. Therefore, as illustrated in Figs. 1 and 2, the connector portion 210 connected to the coupling portion 220 is disposed on the outer surface 610b side of the first band body 610 together with a part of the coupling portion 220.

The slit 615 can be configured to extend, for example, by a predetermined length from the first end 611 of the first band body 610 along the longitudinal direction of the first band body 610.

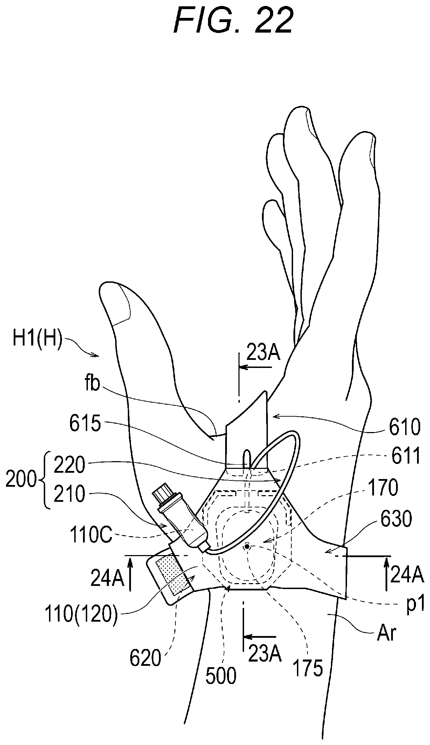

As illustrated in Fig. 22, the first band body 610 can be disposed so as to be hooked on an interdigital portion fb located between the thumb and the index finger of the right hand H1 of the patient in a state where the inflatable member 170 is disposed at the first puncture site p1.

As illustrated in Figs. 1, 2, 3, and 4, the second band body 620 includes a first end 621 integrally connected to the cover main body 110 of the cover member 100, a second end 622 located opposite to the first end 621, and an intermediate portion 623 extending between the first end 621 and the second end 622.

The second direction in which the second band body 620 extends is not particularly limited as long as it is a direction different from the first direction in which the first band body 610 extends.

As illustrated in Figs. 1, 2, 3, and 4, the third band body 630 includes a first end 631 integrally connected to the cover main body 110 of the cover member 100, a second end 632 located opposite to the first end 631, and an intermediate portion 633 extending between the first end 631 and the second end 632.

The third direction in which the third band body 630 extends is not particularly limited as long as it is different from the first direction in which the first band body 610 extends and the second direction in which the second band body 620 extends.

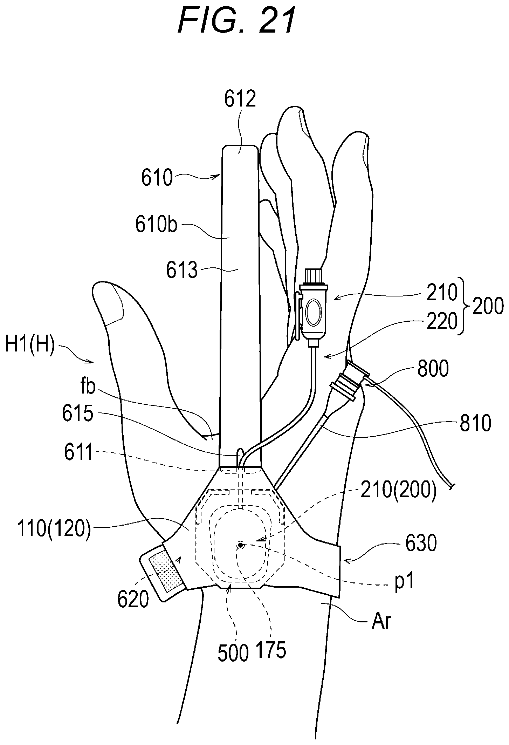

As illustrated in Figs. 21 and 22, the second band body 620 and the third band body 630 can be disposed so as to be wrapped along the outer periphery of the right hand H1 when the hemostatic device 10 is worn on the right hand H1 of the patient.

In plan view illustrated in Fig. 5, the second band body 620 has a flexible portion 625 located closer to the first band body 610 than an end of the first secured region 150A located on the curved region 133 side. Furthermore, in a plan view illustrated in Fig. 5, the third band body 630 has a flexible portion 635 located closer to the first band body 610 than an end of the second secured region 150B located on the curved region 133 side.

Each of the flexible portions 625 and 635 is constituted by a portion where a member forming each of the band bodies 620 and 630 is curved so as to undulate in the thickness direction of each of the band bodies 620 and 630. Each of the flexible portions 625 and 635 is formed to easily extend along a direction in which a tensile force, which is externally applied, is applied as compared with the portions of the band bodies 620 and 630 where the flexible portions 625 and 635 are not formed.

The constituent material of each of the band bodies 610, 620, and 630 is not particularly limited, and each of the band bodies 610, 620, and 630 can be made of, for example, a vinyl chloride resin, a polyurethane resin, a polyester resin, or the like. Furthermore, a shape, a length, a thickness, and the like of each of the band bodies 610, 620, and 630 are not particularly limited.

Four securing portions, that is, a first securing portion 710, a second securing portion 720, a third securing portion 730, and a fourth securing portion 740, which enable the cover member 100 to be secured to the right hand H1, are disposed in the band bodies 610, 620, and 630, respectively.

As illustrated in Fig. 1, the first securing portion 710 is disposed on an outer surface of the intermediate portion 623 of the second band body 620.

As illustrated in Fig. 1, the second securing portion 720 is disposed on an outer surface of the intermediate portion 633 of the third band body 630.

As illustrated in Fig. 2, the third securing portion 730 is disposed on an inner surface of the intermediate portion 633 of the third band body 630.

As illustrated in Fig. 2, the fourth securing portion 740 is disposed on an inner surface of the intermediate portion 613 of the first band body 610.

The first securing portion 710 and the second securing portion 720 can be constituted by, for example, a male side of a hook-and-loop fastener. The third securing portion 730 and the fourth securing portion 740 can be constituted by, for example, a female side of the hook-and-loop fastener. The hook-and-loop fastener in the present specification is a fastener that allows surfaces to be attached and detached, and is, for example, Magic Tape (registered trademark) or Velcro (registered trademark).

The second band body 620 and the third band body 630 are configured to be attachable and detachable by means of the first securing portion 710 located on the outer surface of the intermediate portion 623 of the second band body 620 and the third securing portion 730 located on the inner surface of the intermediate portion 633 of the third band body 630. Furthermore, the first band body 610 and the third band body 630 are configured to be attachable and detachable by means of the fourth securing portion 740 located on the inner surface of the intermediate portion 613 of the first band body 610 and the second securing portion 720 located on the outer surface of the intermediate portion 633 of the third band body 630.

Note that a specific structure of each of the securing portions 710, 720, 730, and 740 is not limited as long as the cover member 100 can be secured to the right hand H1 by connecting the band bodies 610, 620, and 630 to each other in a state where the hemostatic device 10 is disposed on the right hand H1. For example, it is possible to freely eliminate some of the securing portions or to freely change the position where the securing portion is disposed in each of the band bodies 610, 620, and 630. Furthermore, in a case where each of the securing portions 710, 720, 730, and 740 is formed of the hook-and-loop fastener, the male side and the female side of the hook-and-loop fastener may be interchanged. Furthermore, the securing portions 710, 720, 730, and 740 may be configured using, for example, a coupling mechanism or the like including a frame portion formed with a snap, a button, a clip, or a protrusion and an engaged portion formed with a hole engageable with the frame portion.

### (Support Member 500)

As illustrated in Figs. 6 and 7, the support member 500 can be disposed in the insertion portion 135 of the holding portion 130.

As illustrated in Figs. 5, 6, and 7, the support member 500 includes the upper end portion 501 that is located on the first band body 610 side and forms an end of the support member 500, the lower end portion 502 forming an end opposite to the upper end portion 501, and a pair of side surface portions 503 and 504 connecting the upper end portion 501 and the lower end portion 502.

The upper end portion 501 of the support member 500 can be disposed on the fingertip side (distal side) of the finger in a state where the hemostatic device 10 is worn on the right hand H1 of the patient. The lower end portion 502 of the support member 500 can be disposed on the forearm Ar side (proximal side) in a state where the hemostatic device 10 is worn on the right hand H1 of the patient. Note that the upper end portion 501 forms a "distal portion" of the support member 500, and the lower end portion 502 forms a "proximal portion" of the support member 500.

Each of the upper end portion 501 of the support member 500 and the lower end portion 502 of the support member 500 can be configured to include a flat portion extending substantially linearly in a plan view illustrated in Fig. 4.

As illustrated in Fig. 7, the upper surface 500b of the support member 500 is curved toward the second band body 620 and the third band body 630 (the lateral direction in Fig. 7).

A radius of curvature of the upper surface 500b of the support member 500 can be formed to be substantially constant, for example, at each portion in a longitudinal direction (the vertical direction in Fig. 5) of the support member 500.

As illustrated in Fig. 7, the lower surface 500a of the support member 500 is curved toward the second band body 620 and the third band body 630 (the lateral direction in Fig. 7). Therefore, the lower surface 500a of the support member 500 is curved to project in a direction away from the inflatable member 170 as illustrated in Fig. 7.

As illustrated in Fig. 6, the support member 500 can be configured such that the thickness of each of the side surface portions 503 and 504 coupling the upper end portion 501 and the lower end portion 502 decreases from the upper end portion 501 to the lower end portion 502. That is, the support member 500 can be configured to have a cross-sectional shape inclined such that a distance between the upper surface 500b and the lower surface 500a decreases from the upper end portion 501 to the lower end portion 502 in the cross-sectional view illustrated in Fig. 6 and the cross-sectional view illustrated in Fig. 23.

In a plan view illustrated in Fig. 5, the edge portions located at the four corners of the support member 500 can be formed in a rounded curved shape.

The hemostatic device 10 is preferably formed such that a portion of the inflatable member 170 where the marker portion 175 is provided, a portion of the cover member 100 (the upper surface region 131 and the lower surface region 132 of the holding portion 130) overlapping the marker portion 175, and a portion of the support member 500 overlapping the marker portion 175 are transparent. In a case where each of the members 100, 170, and 500 is configured in this manner, an operator can easily visually confirm the position of the marker portion 175 provided on the inflatable member 170 and/or the position of the first puncture site p1 through the portions formed to be transparent in the members 100, 170, and 500 when the hemostatic device 10 is worn on the right hand H1 of the patient as illustrated in Figs. 20, 21, and 22. Note that the term "transparent" in the present specification includes being colored transparent, being colorless transparent, and being translucent.

In a case where the inflatable member 170 is made of the above-described material, examples of a constituent material of the support member 500 include acrylic resin, polyvinyl chloride (particularly hard polyvinyl chloride), polyolefin such as polyethylene, polypropylene, or polybutadiene, polystyrene, poly-(4-methylpentene-1), polycarbonate, ABS resin, polymethyl methacrylate (PMMA), polyacetal, polyacrylate, polyacrylonitrile, polyvinylidene fluoride, ionomer, acrylonitrile-butadiene-styrene copolymer, and polyethylene terephthalate (PET).

### (Injection Member 200)

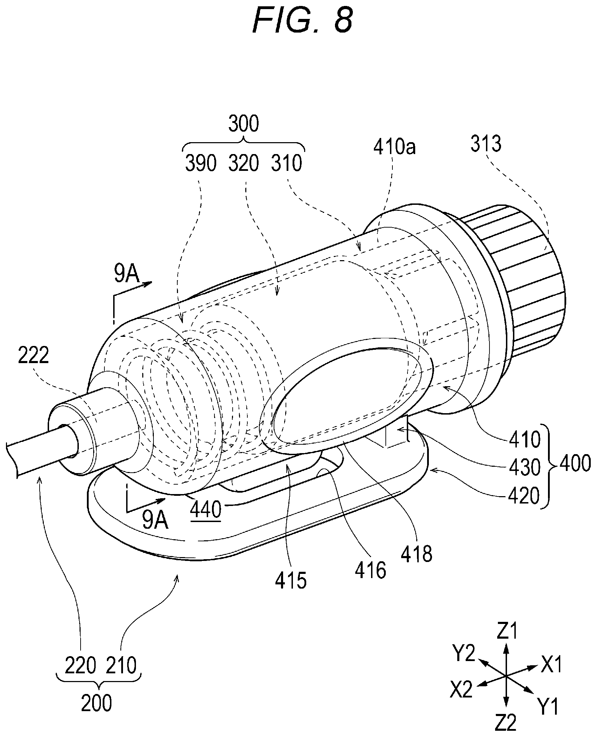

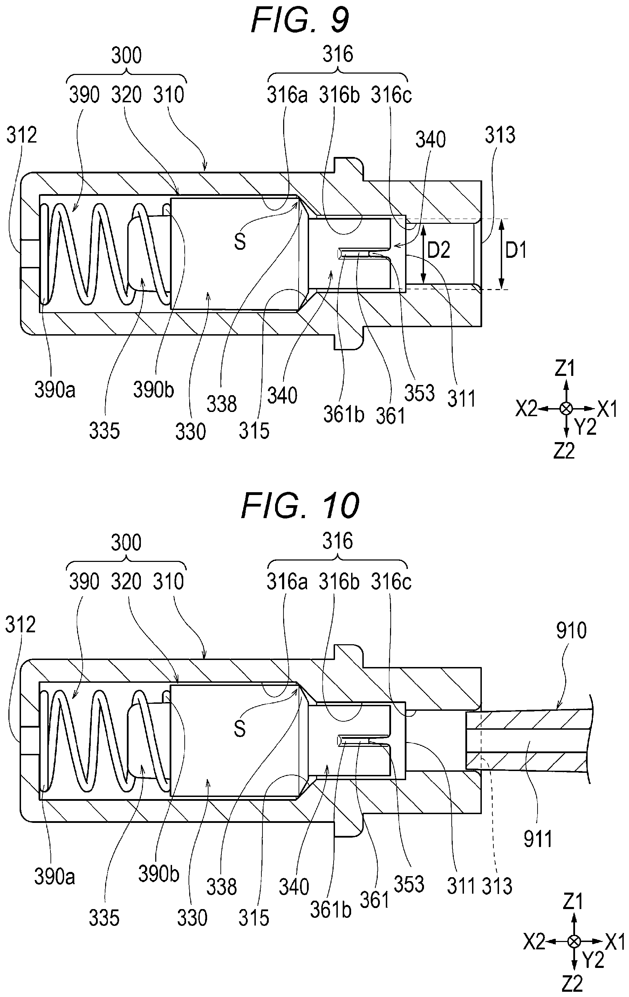

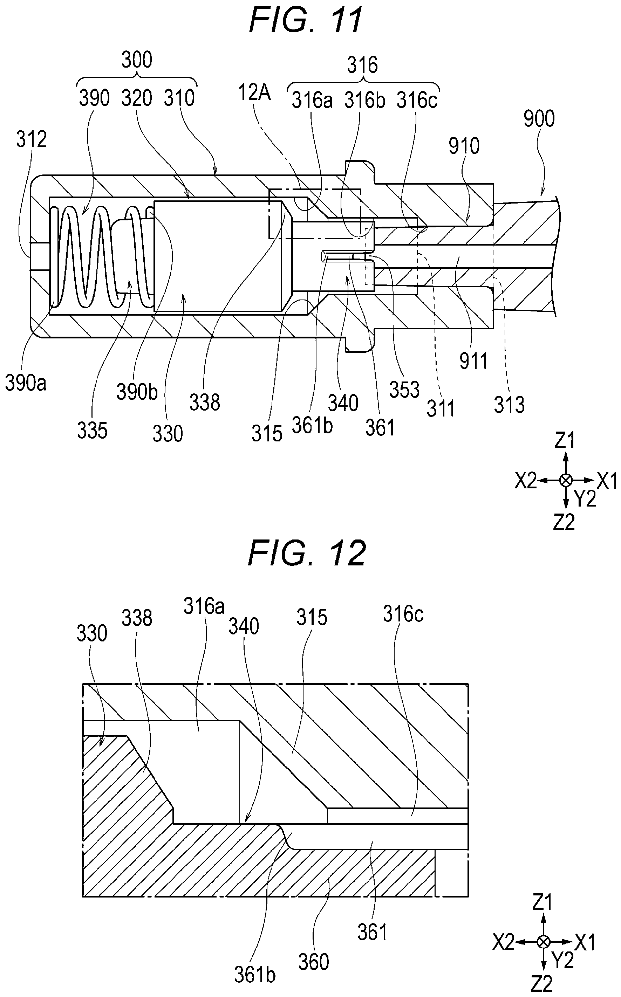

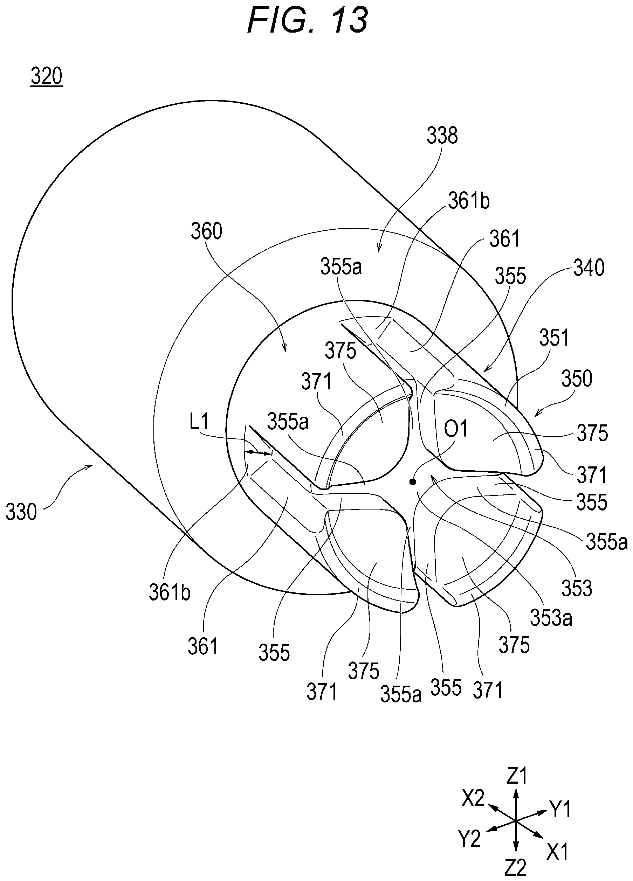

Fig. 8 is a perspective view illustrating the overall configuration of the injection member 200. Figs. 9 to 11 are partial cross-sectional views of a check valve member 300 taken along arrow 9A-9A illustrated in Fig. 8, and Fig. 12 is an enlarged view illustrating a one-dot chain line portion 12A of Fig. 11 together with a cross-sectional view of an inner member 320. Figs. 13 to 18 are views illustrating the inner member 320 included in the check valve member 300.

As illustrated in Figs. 1, 2, and 8, the injection member 200 includes the connector portion 210 and a coupling portion 220 that couples the inner cavity of the connector portion 210 (an inner cavity 410a of a connection member 400 and an inner cavity 316 of an outer member 310 to be described later) and the inner cavity 170a of the inflatable member 170.

The coupling portion 220 is formed of a tube having an inner cavity. The tube constituting the coupling portion 220 can be made of, for example, polyvinyl chloride (in particular, soft polyvinyl chloride), polybutadiene, polyurethane, silicone, or the like.

As illustrated in Fig. 5, one end 221 of the coupling portion 220 is coupled to the inner cavity 170a of the inflatable member 170. As illustrated in Fig. 8, the other end 222 of the coupling portion 220 is coupled to the inner cavity of the connector portion 210.

In the present specification, each direction regarding the connector portion 210 is defined as follows. In the connector portion 210, a side on which an insertion port 313 of the outer member 310 is disposed is referred to as a "distal side (side indicated by arrow X1)", and a side on which the other end 222 of the coupling portion 220 is coupled in the connector portion 210 is referred to as a "proximal side (side indicated by arrow X2)". Furthermore, arrows X1 and X2 in each drawing indicate the longitudinal direction of the connector portion 210, arrows Y1 and Y2 indicate the width direction orthogonal to the longitudinal direction of the connector portion 210, and arrows Z1 and Z2 indicate the height direction of the connector portion 210.

As illustrated in Figs. 8 and 9, the connector portion 210 includes a check valve member 300 configured to be capable of controlling communication between the inner cavity of the connector portion 210 and the outside, and a connection member 400 connecting the check valve member 300 and the coupling portion 220.

The connection member 400 includes an accommodation portion 410 having the inner cavity 410a that accommodates the check valve member 300, a claw portion 420 that extends toward the inflatable member 170 side (the other end side of the coupling portion 220) while facing the accommodation portion 410, and a connection portion 430 that connects the accommodation portion 410 and the claw portion 420.

As illustrated in Fig. 8, a gap portion 440 into which the cover member 100 can be inserted is provided between the accommodation portion 410 and the claw portion 420. As illustrated in Fig. 22, the operator can secure the connector portion 210 and the coupling portion 220 to the cover member 100 by inserting the cover member 100 into the gap portion 440.

As illustrated in Fig. 8, a securing projection 415 for increasing a securing force to the cover member 100 inserted into the gap portion 440 can be provided in a portion of the accommodation portion 410 facing the claw portion 420. Furthermore, the claw portion 420 can be provided with a hole 416 at a position facing the securing projection 415. When disposing the cover member 100 in the gap portion 440, the operator can smoothly insert the cover member 100 into the gap portion 440 by moving a part of the cover member 100 toward the hole 416 so as to recede therein while curving the part of the cover member 100 along the securing projection 415. Furthermore, in a state where the cover member 100 is inserted into the gap portion 440, the hemostatic device 10 can hold a part of the cover member 100 in a meandering state between the securing projection 415 and the hole 416. Thus, the hemostatic device 10 can increase the securing force of the connector portion 210 with respect to the cover member 100.

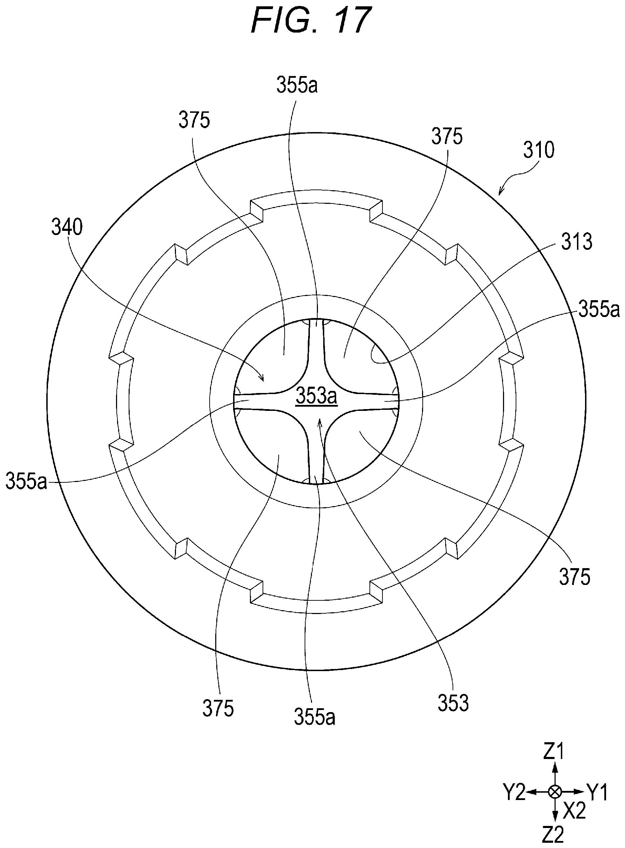

As illustrated in Figs. 8 and 17, a part of the distal portion where the insertion port 313 of the outer member 310 is located protrudes from the distal portion of the accommodation portion 410.

When inflating the inflatable member 170, the operator inserts a distal tubular portion 910 of a fluid injection device 900 into the inner cavity 410a of the connection member 400 and the inner cavity 316 of the outer member 310 via the insertion port 313 of the outer member 310. Furthermore, as illustrated in Figs. 10 and 11, in a state where the distal tubular portion 910 is inserted into the inner cavity 316 of the outer member 310 via the insertion port 313, the operator can further move the distal tubular portion 910 toward the proximal side to push the distal tubular portion 910 to a position closer to the proximal side than the distal opening 311 of the outer member 310.

As illustrated in Figs. 8 and 9, the check valve member 300 includes an outer member 310, an inner member 320 located in the inner cavity 316 of the outer member 310, and a spring member 390 connected to the inner member 320.

As illustrated in Fig. 9, the outer member 310 includes a distal opening 311 configured to expose a part of the inner member 320 to the outside, a proximal opening 312 that communicates the inner cavity 316 of the outer member 310 and the inner cavity 410a of the connection member 400, and an inner protrusion portion 315 configured to prevent the inner member 320 from moving toward the distal opening 311.

In a plan view illustrated in Fig. 17 (plan view of the connector portion 210 as viewed from the proximal side), a part of the distal protrusion portion 340 of the inner member 320 is exposed to the outside of the connector portion 210 through the distal opening 311 and the insertion port 313 (see Fig. 9) of the outer member 310.

As illustrated in Fig. 9, in the inner cavity 316 of the outer member 310, a first portion 316a in which the main body 330 of the inner member 320 is disposed, a second portion 316b located on the distal side relative to the first portion 316a and having an inner diameter smaller than that of the first portion 316a, and a third portion 316c located on the distal side relative to the second portion 316b and having an inner diameter smaller than that of the second portion 316b are formed.

The inner protrusion portion 315 is located at the distal portion of the first portion 316a. The distal portion of the first portion 316a has a tapered shape in which the inner diameter gradually decreases toward the second portion 316b side (distal side).

As illustrated in Figs. 9 and 10, the spring member 390 is located between the proximal opening 312 and the inner protrusion portion 315 so as to maintain contact between the outer surface of the inner member 320 and the inner protrusion portion 315 in a natural state.

The above-described "natural state" means a state where a force in a compressing direction is not applied to the spring member 390 (a state where the distal tubular portion 910 of the fluid injection device 900 is not pressed against the inner member 320).

The proximal portion 390a of the spring member 390 is secured to the inner wall of the proximal portion of the outer member 310. Furthermore, the distal portion 390b of the spring member 390 is connected to the proximal portion 335 of the inner member 320.

The inner member 320 includes a main body 330 configured to be in contact with the inner protrusion portion 315 in a natural state, and a distal protrusion portion 340 that protrudes from the distal end of the main body 330 toward the distal opening 311 side.

The inner member 320 has a proximal portion 335 located on the proximal side relative to the main body 330. The proximal portion 335 has an outer shape smaller than that of the main body 330. The proximal portion 335 is inserted into a space extending inside the spring member 390.

The distal portion of the main body 330 has a tapered shape in which the outer shape size gradually decreases toward the distal protrusion portion 340 side. The distal portion of the main body 330 constitutes a seal-forming portion 338 that maintains contact with the inner protrusion portion 315 when the spring member 390 is in a natural state.

As illustrated in Figs. 9 and 10, the spring member 390 brings the outer surface of the seal-forming portion 338 of the main body 330 into contact with the inner protrusion portion 315 in a natural state to form a seal portion S in the vicinity of the inner protrusion portion 315. Since the seal portion S is formed in the inner cavity 316 of the outer member 310, the connector portion 210 prevents fluid communication between the inner cavity 170a of the inflatable member 170 and the outside of the connector portion 210 via the coupling portion 220 and the connector portion 210.

As shown in Fig. 11, the spring member 390 separates the outer surface of the seal-forming portion 338 of the main body 330 from the inner protrusion portion 315 when the inner member 320 is pushed toward the proximal side by the distal tubular portion 910 of the fluid injection device 900 inserted into the inner cavity 316 of the outer member 310. When the outer surface of the seal-forming portion 338 of the main body 330 is separated from the inner protrusion portion 315, an unsealed state is obtained in which the seal portion S is not formed in the inner cavity 316 of the outer member 310. The hemostatic device 10 can cause the inner cavity 170a of the inflatable member 170 and the inner cavity 911 of the distal tubular portion 910 to be in fluid communication with each other by bringing the inner cavity 316 of the outer member 310 into the unsealed state. The operator can inject the fluid into the inner cavity 170a of the inflatable member 170 by operating the fluid injection device 900 in a state where the inner cavity 170a of the inflatable member 170 and the inner cavity 911 of the distal tubular portion 910 are in fluid communication. When releasing the fluid communication between the inner cavity 170a of the inflatable member 170 and the inner cavity 911 of the distal tubular portion 910, the operator withdraws the distal tubular portion 910 from the connector portion 210 (the outer member 310 and the connection member 400). In the hemostatic device 10, when the distal tubular portion 910 is withdrawn from the connector portion 210, the spring member 390 is restored to a natural state. The spring member 390 is restored to a natural state brings the outer surface of the seal-forming portion 338 of the main body 330 into contact with the inner protrusion portion 315 to form the seal portion S in the vicinity of the inner protrusion portion 315.

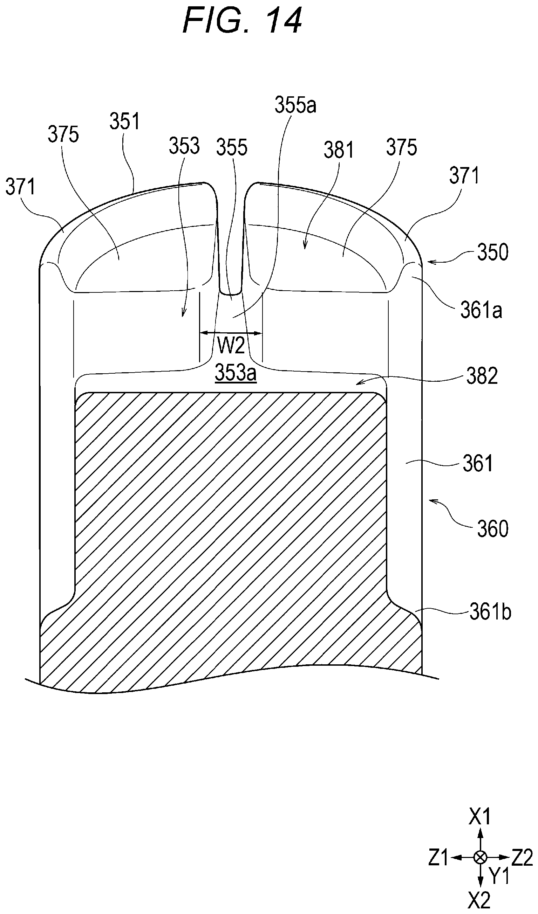

As illustrated in Figs. 13 and 14, the distal protrusion portion 340 includes an upper end portion 350 and a side wall portion 360 connecting the upper end portion 350 and the main body 330.

The upper end portion 350 is an end portion located on the distal side (the insertion port 313 side of the outer member 310) of the distal protrusion portion 340.

The distal protrusion portion 340 has an outer shape smaller than that of the main body 330. In the present embodiment, the main body 330 has a substantially cylindrical outer shape, and the distal protrusion portion 340 also has a substantially cylindrical outer shape. Therefore, the distal protrusion portion 340 is configured to have a smaller outer diameter than that of the main body 330.

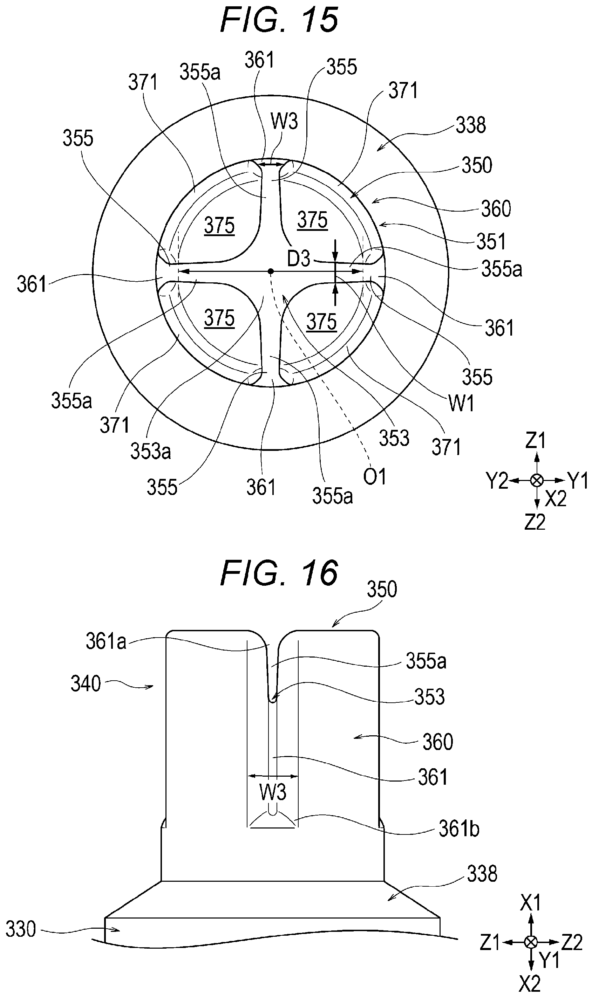

The side wall portion 360 extends substantially linearly between the main body 330 and the upper end portion 350 in a side view illustrated in Fig. 16.

As illustrated in Figs. 13 and 14, the upper end portion 350 has a recess portion 353 recessed toward the main body 330 side (proximal side).

As illustrated in Figs. 13, 14, and 16, the side wall portion 360 has a slit portion 361 which is continuous with the recess portion 353 and extends in the longitudinal direction of the inner member 320.

The recess portion 353 has a trap portion 355 having a width W1 that narrows from a center position O1 of the upper end portion 350 toward the outer edge 351 of the upper end portion 350 in a plan view illustrated in Fig. 15.

In the present embodiment, in the plan view illustrated in Fig. 15, the recess portion 353 extending in a substantially symmetrical shape in the vertical direction (directions of arrows Z1 and Z2) and the lateral direction (directions of arrows Y1 and Y2) with respect to the center position O1 of the upper end portion 350 is formed in the upper end portion 350.

Furthermore, in the present embodiment, in the plan view illustrated in Fig. 15, the trap portions 355, in which the width W1 is formed narrower than the portion located at the center position O1 of the recess portion 353, are formed at four positions near the upper end portion, the lower end portion, the left end portion, and the right end portion of the recess portion 353 extending in a cutout shape. Note that in the present embodiment, the "center position O1 of the recess portion 353" means the same position as the "center position O1 of the upper end portion 350".

As illustrated in Fig. 14, the recess portion 353 is connected to the slit portion 361 via the trap portion 355. That is, the recess portion 353 communicates with the outer surface side of the side wall portion 360 from the center position O1 of the upper end portion 350 via the trap portion 355 and the slit portion 361.

In the hemostatic device 10, four trap portions 355 are formed in the recess portion 353. The slit portions 361 are formed at positions substantially the same as the circumferential positions where the trap portions 355 are disposed, one by one (four in total), so as to correspond to the number and positions of the trap portions 355.

The hemostatic device 10 can catch a foreign matter having entered the inner cavity of the connector portion 210 (the inner cavity 316 of the outer member 310 located in the inner cavity 410a of the connection member 400) by the trap portion 355 formed in the upper end portion 350 included in the inner member 320. The foreign matter to be captured by the trap portion 355 is not particularly limited, and is, for example, the fragment of the hook-and-loop fastener used for each of the securing portions 710, 720, 730, and 740 of the hemostatic device 10. Even in a case where the foreign matter enters the inner cavity of the connector portion 210, the hemostatic device 10 can capture the foreign matter by the trap portion 355 at a position closer to the distal side (side on which the insertion port 313 and the distal opening 311 serving as a path through which the foreign matter enters are located when viewed from the inner protrusion portion 315) than the inner protrusion portion 315 forming the seal portion S. Therefore, the hemostatic device 10 can prevent the foreign matter having entered the inner cavity of the connector portion 210 from moving to the position where the inner protrusion portion 315 in the inner cavity 316 of the outer member 310 is located. Thus, the hemostatic device 10 can prevent the foreign matter from being caught between the outer surface of the main body 330 of the inner member 320 and the inner protrusion portion 315 even in a case where the foreign matter enters the inner cavity of the connector portion 210. Thus, the hemostatic device 10 can prevent the sealing performance of the seal portion S formed in the inner cavity 316 of the outer member 310 from being degraded due to the influence of the foreign matter entering the inner cavity of the connector portion 210.

Furthermore, as illustrated in Figs. 11 and 12, when the fluid is injected into the inner cavity of the connector portion 210 via the distal tubular portion 910 of the fluid injection device 900 in the unsealed state where the seal-forming portion 338 of the main body 330 is separated from the inner protrusion portion 315, the hemostatic device 10 can smoothly move the injected fluid from the distal side toward the proximal side of the connector portion 210 via the recess portion 353 and the slit portion 361 connected to the recess portion 353. Therefore, when the operator operates the fluid injection device 900 to inject the fluid into the inner cavity 170a of the inflatable member 170, it is possible to reduce the resistance (resistance to pushing) of the hand felt by the operator. Thereby, the operator can smoothly perform the hemostasis at the first puncture site p1 using the hemostatic device 10.

As illustrated in Fig. 16, the slit portion 361 extends along the longitudinal direction of the side wall portion 360 with a substantially constant width W3.

As illustrated in Figs. 13 and 14, the upper end portion 350 has a first projection 371 formed on the outer edge 351 of the upper end portion 350.

The above-described "outer edge 351 of the upper end portion 350" means an arbitrary position on a line represented by connecting a range including a cutout shape portion where the trap portion 355 is formed with an imaginary line when the upper end portion 350 is viewed in the plan view illustrated in Fig. 15. In the present embodiment, the planar shape of the outer edge 351 of the upper end portion 350 is represented by an imaginary circle centered on the center position O1 of the upper end portion 350.

As illustrated in Fig. 14, the first projection 371 protrudes toward the opposite side (distal side) to the side where the main body 330 is located. The end located in the protruding direction of the first projection 371 can be formed to have a rounded curved shape toward the distal side.

As illustrated in Figs. 13 and 15, the upper end portion 350 has a plurality of upper surface portions 375 located on the same plane.

Four upper surface portions 375 are formed at different positions in the circumferential direction with respect to the center position O1 of the upper end portion 350. In the plan view illustrated in Fig. 15, the trap portion 355 is located between the upper surface portions 375 adjacent to each other in the circumferential direction of the upper end portion 350.

The upper surface portions 375 are located at a position separated from the main body 330 by a predetermined distance further than the bottom portion 353a of the recess portion 353. Each of the upper surface portions 375 has a flat surface shape extending substantially in parallel along a direction orthogonal to the extending direction of the upper end portion 350. The first projection 371 is located at the outer edge 351 of the upper end portion 350 located on the outer edge side (the side separated from the center position O1 in the plan view illustrated in Fig. 15) relative to the upper surface portion 375. The upper surface portion 375 is located on the main body 330 side relative to an end (distal portion) located in the protruding direction of the first projection 371.

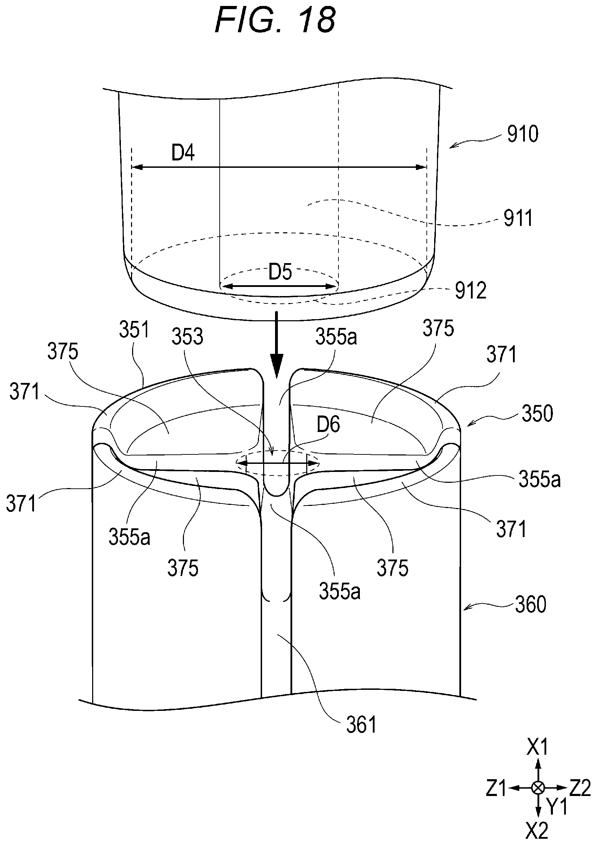

When the operator injects the fluid into the inner cavity 170a of the inflatable member 170 using the fluid injection device 900, the distal surface of the distal tubular portion 910 included in the fluid injection device 900 can be disposed to be in contact with each of the upper surface portions 375 having a flat surface shape extending substantially parallel along a direction orthogonal to the extending direction of the upper end portion 350 with the greater area (see Fig. 18).

As illustrated in Fig. 9, the outer diameter D1 of the upper end portion 350 can be configured to be greater than the inner diameter D2 of the distal opening 311 of the outer member 310.

Since the outer diameter D1 of the upper end portion 350 is greater than the inner diameter D2 of the distal opening 311 of the outer member 310, the hemostatic device 10 is disposed such that a certain range from the outer edge 351 of the upper end portion 350 toward the center position O1 overlaps the distal portion of the outer member 310 (a portion located on the distal side relative to the distal opening 311) in a state where the inner member 320 is disposed in the inner cavity 316 of the outer member 310 as illustrated in Fig. 9. Therefore, as illustrated in a plan view of Fig. 17, when the inner cavity 316 of the outer member 310 is viewed from the distal side of the outer member 310 (distal side of the connection member 400), the range (portion) of the inner member 320 exposed through the insertion port 313 of the outer member 310 is limited to a certain range including the center position O1 of the distal protrusion portion 340 and the upper surface portion 375.

In the natural state illustrated in Figs. 9 and 10, the spring member 390 holds the other end 361b of the slit portion 361 located on the main body 330 side at a position on the distal opening 311 side relative to the inner protrusion portion 315.

Note that the other end 361b of the slit portion 361 is an end located closest to the main body 330 in the slit portion 361. One end 361a of the slit portion 361 is an end located on the opposite side (distal side) to the other end 361b.

As illustrated in Figs. 11 and 12, the spring member 390 is configured to move the other end 361b of the slit portion 361 to a position on the proximal opening 312 side relative to the inner protrusion portion 315 when a pushing force in a direction toward the proximal opening 312 is applied to the spring member 390. Note that in the spring member 390, the length in the natural state, the amount of expansion/contraction deformation, the elastic modulus, and the like can be arbitrarily set such that the other end 361b of the slit portion 361 can be disposed at each position illustrated in Figs. 9 to 11 in the natural state and the state where the pushing force is applied.

As illustrated in Figs. 13, 14, and 15, the recess portion 353 has a cutout portion 355a connecting the center position O1 of the upper end portion 350 and a side surface (outer peripheral surface) of the upper end portion 350.

The cutout portion 355a of the recess portion 353 is configured by a cutout shape space formed in the longitudinal direction of the distal protrusion portion 340. In the recess portion 353, the center position O1 of the upper end portion 350 and the side surface of the upper end portion 350 are connected (communicated) via the cutout portion 355a located between the center position O1 of the upper end portion 350 and the side surface of the upper end portion 350 facing the outside. Note that the trap portion 355 is constituted by a part of the cutout portion 355a located in the vicinity of the outer edge 351 of the upper end portion 350.

As illustrated in Fig. 14, the width W2 of the cutout portion 355a gradually decreases toward the main body 330 side (downward in the drawing).

As illustrated in Figs. 13 and 15, the cutout portions 355a can be disposed at four locations at equal intervals in the circumferential direction of the upper end portion 350 with reference to the center position O1 of the upper end portion 350. A circumferential angle difference between the cutout portions 355a is approximately 90°.

As illustrated in Fig. 13, the slit portion 361 has a predetermined depth L1 along a direction from the side wall portion 360 side toward the center position O1 side of the upper end portion 350. The depth L1 of the slit portion 361 is preferably, for example, 0.25 mm or more and 0.35 mm or less. Furthermore, the outer diameter of the distal protrusion portion 340 is preferably, for example, 3.25 mm or more and 3.35 mm or less.

As illustrated in Fig. 14, the upper end portion 350 includes a first region 381 including the upper surface portion 375 and a second region 382 forming the bottom portion 353a of the recess portion 353.

As illustrated in Fig. 14, the other end 361b of the slit portion 361 located on the main body 330 side is located on the main body 330 side relative to the second region 382.

In the present embodiment, the hemostatic device 10 and the fluid injection device 900 (see Fig. 18) including the distal tubular portion 910 configured to abut the upper end portion 350 of the inner member 320 (in the present embodiment, the upper surface portion 375 included in the upper end portion 350 of the inner member 320) can constitute a hemostatic device assembly (corresponding to a "hemostatic device set") used for hemostasis at the first puncture site p1 formed on the right hand H1 of the patient.

The fluid injection device 900 can be configured by, for example, a known fluid supply syringe in the medical field.

In a case where the hemostatic device assembly is constituted by the hemostatic device 10 and the fluid injection device 900 as described above, the upper surface portion 375 that the distal tubular portion 910 of the fluid injection device 900 abuts in the upper end portion 350 of the inner member 320, can be configured to have an outer diameter greater than that of the distal tubular portion 910 in a plan view. That is, the outer diameter D3 of the upper surface portion 375 illustrated in Fig. 15 can be made greater than the outer diameter D4 (the outer diameter of the most distal portion) of the distal tubular portion 910 illustrated in Fig. 18.

The above-described "outer diameter D3 of the upper surface portion 375" means an outer diameter of an imaginary circle represented by connecting the outer periphery portion of the upper surface portion 375 including a cutout shape portion in which the trap portion 355 is formed using an imaginary line when the upper end portion 350 is viewed in the plan view illustrated in Fig. 15.

It is preferable that the inner diameter D5 of the injection port 912 included in the distal tubular portion 910 is smaller than the outer diameter D6 of the inner peripheral portion of the recess portion 353 (inner diameter D5 < outer diameter D6) (see Fig. 18).

The above-described "outer diameter D6 of the inner peripheral portion of the recess portion 353" can be defined by an outer diameter of an imaginary circle connecting end portions of the upper surface portions 375 located at positions closest to the center position O1 side of the upper end portion 350.

For example, when the inner diameter D5 of the injection port 912 is greater than the outer diameter D6 of the inner peripheral portion of the recess portion 353, it is conceivable that the fluid is blown to the foreign matter existing on the upper surface portion 375 when the fluid is injected through the injection port 912 in a state where the foreign matter exists on the upper surface portion 375. Thus, there is a concern that the foreign matter moves from the upper surface portion 375 to the main body 330 side via the slit portion 361 and the like, which leads to degradation of the sealing performance of the seal portion S. When the inner diameter D5 of the injection port 912 included in the distal tubular portion 910 is equal to or less than the outer diameter D6 of the inner peripheral portion of the recess portion 353, it is possible to prevent the fluid injected from the injection port 912 from being directly blown to the foreign matter existing on the upper surface portion 375. Thus, it is possible to prevent in advance degradation in sealing performance of the seal portion S due to the influence of the foreign matter as described above.

The connection member 400 and the outer member 310 can be formed of, for example, an ABS resin or a polypropylene resin.

The inner member 320 can be formed of an elastic member such that a predetermined sealing performance can be exerted by the outer surface of the main body 330 coming into contact with the inner protrusion portion 315 when the spring member 390 is in a natural state. As a constituent material of the inner member 320 exhibiting the above-described function, for example, silicone rubber, ethylene propylene diene rubber, or the like can be used. Note that the inner member 320 is preferably colored in green or the like such that a state where the foreign matter adheres to the inner member 320 or the foreign matter is placed can be easily visually confirmed.

### <Usage Example of Hemostatic Device>

Next, the usage example of the hemostatic device 10 will be described with reference to Figs. 20 to 24. Hereinafter, a process of using the hemostatic device 10 to perform hemostasis at the first puncture site p1 formed on the right hand H1 of the patient illustrated in Fig. 19 will be described.

Fig. 20 illustrates a state where various procedures performed using the sheath tube 810 of the introducer 800 inserted into the first puncture site p1 are completed.

When the hemostatic device 10 is worn on the right hand H1 of the patient, the operator places the inflatable member 170 and the support member 500 so as to overlap the first puncture site p1 as illustrated in Fig. 20. At this time, the operator can appropriately position the inflatable member 170 and the support member 500 at the first puncture site p1 by disposing the marker portion 175 at the first puncture site p1 while visually confirming the position of the marker portion 175 provided on the inflatable member 170.

When the hemostatic device 10 is worn on the right hand H1 of the patient, the operator can dispose the support member 500 so as to overlap at least a part of the metacarpal bone B1 of the index finger and at least a part of the metacarpal bone B2 of the thumb. When, for example, performing hemostasis at the first puncture site p1, the operator can dispose the support member 500 on a predetermined position P1 located on the extension of the metacarpal bone B1 and the metacarpal bone B2 as illustrated in Fig. 19. Furthermore, when performing hemostasis at the second puncture site p2, the operator can dispose the support member 500 at a predetermined position P2 located on the extension of the metacarpal bone B1 and the metacarpal bone B2 and on the forearm Ar side relative to the predetermined position P1 as illustrated in Fig. 19.

As illustrated in Fig. 21, the operator wraps the second band body 620 and the third band body 630 along the outer periphery of the right hand H1 of the patient.

The operator can connect the second band body 620 and the third band body 630 by means of the securing portions 710 and 730 by bringing the third securing portion 730 (see Fig. 2) disposed on the inner surface of the third band body 630 into contact with the first securing portion 710 (see Fig. 1) disposed on the outer surface of the second band body 620. The operator can firmly wrap the band bodies 620 and 630 along the outer periphery of the right hand H1 by connecting the band bodies 620 and 630 to each other in a state where the support member 500 overlaps at least a part of the metacarpal bone B1 of the index finger and at least a part of the metacarpal bone B2 of the thumb.

The upper surface 500b of the support member 500 is curved toward the second band body 620 (toward the side surface portion 503) and the third band body 630 (toward the side surface portion 504) (see Fig. 7). Therefore, the operator can push the support member 500 against the right hand H1 of the patient from the upper surface 500b side along the outer periphery of the right hand H1 as illustrated in Fig. 24 by wrapping the second band body 620 and the third band body 630 along the outer periphery of the right hand H1 as illustrated in Fig. 21. Therefore, the hemostatic device 10 can increase the securing force for securing the support member 500 and the inflatable member 170 to the right hand H1 of the patient.

As illustrated in Fig. 22, the operator disposes a part of the first band body 610 on a palm side of the right hand H1 of the patient while passing the first band body 610 through the interdigital portion fb located between the thumb and the index finger of the right hand H1 of the patient. At this time, the operator brings the fourth securing portion 740 (see Fig. 2) disposed on the inner surface of the first band body 610 into contact with the second securing portion 720 (see Fig. 1) disposed on the outer surface of the third band body 630, thereby being capable of connecting the first band body 610 and the third band body 630 by means of the securing portions 720 and 740.

The right hand H1 of the patient has an inclined portion at each location (for example, the vicinity of the snuff box Sb). Furthermore, a shape of the inclined portion of the right hand H1 depends on individual differences of patients. For example, in a case where the lower surface 500a of the support member 500 is formed in a flat shape, it is difficult to dispose the support member 500 along the inclined portion of the right hand H1. In the hemostatic device 10 according to the present embodiment, the lower surface 500a of the support member 500 is curved to project in a direction away from the inflatable member 170 (see Figs. 7 and 24). Therefore, when the lower surface 500a of the support member 500 is disposed so as to overlap the inclined portion of the right hand H1 of the patient, the lower surface 500a of the support member 500 can be disposed along the right hand H1 of the patient. Thus, in the hemostatic device 10, the support member 500 can be disposed so as to overlap the first puncture site p1 without depending on individual difference of the right hand H1 and the left hand of the patient or the way of placement by the operator. Therefore, in the hemostatic device 10, the support member 500 can be appropriately disposed at a desired position near the first puncture site p1.

Moreover, since the lower surface 500a of the support member 500 is disposed along the body surface of the right hand H1 when the operator wraps the second band body 620 and the third band body 630 along the right hand H1 of the patient, the hemostatic device 10 can be disposed such that the second band body 620 and the third band body 630 extending from the side surface portions 503 and 504 of the support member 500 are disposed in proximity to or in close contact with the body surface of the right hand H1 of the patient (see Fig. 24). Therefore, the hemostatic device 10 can increase the securing force for securing the support member 500 and the inflatable member 170 to the right hand H1 of the patient. Thus, even in a case where an impact or the like is applied from the outside in a state where the hemostatic device 10 is worn on the right hand H1 of the patient, the support member 500 and the inflatable member 170 are less likely to be displaced.

The operator connects the fluid injection device 900 to the connector portion 210 in a state where the band bodies 610, 620, and 630 are connected to each other and the hemostatic device 10 is secured to the right hand H1 of the patient. By connecting the fluid injection device 900 to the connector portion 210, the operator moves the inner member 320 disposed in the inner cavity 316 of the outer member 310 to the proximal side, releases the sealed state by the seal portion S formed by the seal-forming portion 338 of the inner member 320 and the inner protrusion portion 315 of the outer member 310, and enters the unsealed state (see Figs. 11 and 12). After bringing the inner cavity of the connector portion 210 (the inner cavity 316 of the outer member 310) into the unsealed state, the operator operates the fluid injection device 900 to inject the fluid into the inner cavity of the connector portion 210. When the operator injects the fluid into the inner cavity of the connector portion 210, the injected fluid is injected into the inner cavity 170a of the inflatable member 170 via the connector portion 210 and the coupling portion 220. The hemostatic device 10 inflates the inflatable member 170 by injecting the fluid into the inner cavity 170a of the inflatable member 170.

As illustrated in Figs. 23 and 24, when the inflatable member 170 is inflated in the hemostatic device 10, the inflatable member 170 applies a compressive force to the first puncture site p1.

After inflating the inflatable member 170 to the desired inflation amount, the operator removes the fluid injection device 900 from the connector portion 210. When the operator removes the fluid injection device 900 from the connector portion 210, the spring member 390 disposed in the inner cavity 316 of the outer member 310 is restored to a natural state (see Figs. 9 and 10). When the spring member 390 is restored to the natural state, the outer surface of the seal-forming portion 338 of the inner member 320 comes into contact with the inner protrusion portion 315 of the outer member 310, and the seal portion S is formed in the inner cavity 316 of the outer member 310. The hemostatic device 10 can maintain the sealing performance of the inner cavity of the connector portion 210 by forming the seal portion S in the inner cavity 316 of the outer member 310. Therefore, the hemostatic device 10 can prevent the fluid injected into the inner cavity 170a of the inflatable member 170 from leaking out of the connector portion 210 through the coupling portion 220 and the inner cavity of the connector portion 210 after the fluid injection device 900 is removed from the connector portion 210.

When the inflatable member 170 is inflated in a state where the hemostatic device 10 is worn on the right hand H1 of the patient, the support member 500 presses the inflatable member 170 against the right hand H1 of the patient. Thus, the hemostatic device 10 can prevent the inflatable member 170 from lifting off the right hand H1 of the patient.

When performing hemostasis using the hemostatic device, the operator can strongly tighten the first band body disposed in the interdigital portion fb, for example, in order to enhance the securing force of securing the support member with respect to the right hand H1 of the patient. By strongly tightening the first band body, the operator can firmly secure the support member to the right hand H1 of the patient even when the support member is disposed in an unstable state on the right hand H1 of the patient. However, when the first band body is strongly tightened, the patient may feel pain. Therefore, in a case where the first band body cannot be strongly tightened due to pain or the like of the patient, the hemostatic device may be disposed such that the lower surface of the support member is inclined with respect to the body surface of the right hand H1 of the patient. Specifically, the upper end portion of the support member on which the first band body is disposed is lifted to a position excessively away from the body surface of the right hand H1 of the patient as compared with the lower end portion located opposite to the upper end portion. Thus, in the hemostatic device, the support member is disposed to be inclined such that a distance between the lower surface of the support member and the body surface of the right hand H1 of the patient gradually decreases from the upper end portion to the lower end portion of the support member.

The hemostatic device 10 is configured such that the thickness of each of the side surface portions 503 and 504 of the support member 500 is decreased from the upper end portion 501 to the lower end portion 502 (see Fig. 6). In addition, the first band body 610 configured to be disposed at the interdigital portion fb located between the fingers of the patient extends from the upper end portion 501 side of the support member 500, and is configured to push the upper end portion 501 side of the support member 500 against the right hand H1 of the patient. In the hemostatic device 10 configured as described above, when the inflatable member 170 starts to be inflated in a state where the support member 500 and the inflatable member 170 are secured to the right hand H1 of the patient, the inflatable member 170 applies a force to the vicinity of the upper end portion 501 of the support member 500. The lower end portion 502 side of the support member 500 is lifted with the upper end portion 501 side being pushed against the right hand H1 by the force applied by the inflatable member 170. Thus, the support member 500 is disposed such that the lower surface 500a of the support member 500 is substantially parallel with the body surface of the right hand H1 of the patient in a state where the inflatable member 170 is inflated as illustrated in Fig. 23. Therefore, the hemostatic device 10 can apply a compressive force in a direction perpendicular to the first puncture site p1 when the inflatable member 170 is inflated.

After the inflatable member 170 is inflated, the operator removes the sheath tube 810 of the introducer 800 from the first puncture site p1 as illustrated in Fig. 22. The operator confirms that there is no bleeding from the first puncture site p1 while performing hemostasis using the hemostatic device 10. In a case where there is bleeding from the first puncture site p1, the operator can appropriately adjust an injection amount of the fluid into the inflatable member 170.

As illustrated in Fig. 22, in a state where the inflatable member 170 is inflated, the operator can secure the connector portion 210 and the coupling portion 220 coupled to the connector portion 210 to the cover member 100 by means of the connection member 400 (see Fig. 8). By securing the connector portion 210 and the coupling portion 220 to the cover main body 110, the operator can suppress inadvertent movement of the coupling portion 220 following the movement of the right hand H1 of the patient while the inflatable member 170 applies the compressive force to the first puncture site p1.

The operator can secure the connector portion 210 to the cover member 100 by inserting a part of the cover member 100 into the gap portion 440 (see Fig. 8) extending between the accommodation portion 410 and the claw portion 420 extending in the longitudinal direction of the connector portion 210. Therefore, when the connector portion 210 is secured to the cover member 100, the hemostatic device 10 can prevent an unnecessary force from being applied in a direction of pushing the hemostatic device 10 against the body surface of the right hand H1 of the patient. Therefore, when the connector portion 210 is secured to the cover member 100, the hemostatic device 10 can prevent the hemostatic device 10 worn on the right hand H1 of the patient from being displaced or the inflatable member 170 from being displaced.

The operator can secure the connector portion 210 at an arbitrary position where a space (gap) in which the claw portion 420 can be disposed is formed between the cover member 100 and the body surface of the right hand H1 when the inflatable member 170 is inflated. As such a position, a secured portion 110C (see Fig. 22) located between the first band body 610 and the second band body 620 in the cover main body 110 can be exemplified. However, the position where the connector portion 210 is secured in the cover member 100 is not limited to the secured portion 110C. For example, the connector portion 210 can be secured to a peripheral portion of the first end 621 of the second band body 620, an arbitrary position of the first band body 610, an arbitrary position of the second band body 620, and the like in the cover main body 110.

Hereinafter, operational effects of the hemostatic device 10 according to the present embodiment will be described.

The hemostatic device 10 includes a cover member 100 configured to cover the first puncture site p1 formed in the patient, an inflatable member 170 connected to the cover member 100 and configured to compress the first puncture site p1, an injection member 200 connected to the inflatable member 170 and configured to be capable of injecting the fluid into the inflatable member 170. The injection member 200 includes a connector portion 210 and a coupling portion 220 that couples the inner cavity of the connector portion 210 and the inner cavity 170a of the inflatable member 170. The connector portion 210 includes a check valve member 300 configured to be capable of controlling communication between the inner cavity of the connector portion 210 and the outside, and a connection member 400 connecting the check valve member 300 and the coupling portion 220. The check valve member 300 includes an outer member 310, an inner member 320 located in the inner cavity 316 of the outer member 310, and a spring member 390 connected to the inner member 320. The outer member 310 includes a distal opening 311 configured to expose a part of the inner member 320 to the outside, a proximal opening 312 that communicates the inner cavity 316 of the outer member 310 and the inner cavity 410a of the connection member 400, and an inner protrusion portion 315 configured to prevent the inner member 320 from moving toward the distal opening 311. The spring member 390 is located between the proximal opening 312 and the inner protrusion portion 315 so as to maintain contact between the outer surface of the inner member 320 and the inner protrusion portion 315 in a natural state, and the inner member 320 includes a main body 330 configured to be in contact with the inner protrusion portion 315 in the natural state, and a distal protrusion portion 340 that protrudes from the distal end of the main body 330 toward the distal opening 311 side. The distal protrusion portion 340 includes an upper end portion 350 and a side wall portion 360 connecting the upper end portion 350 and the main body 330. The upper end portion 350 has a recess portion 353 recessed toward the main body 330 side, and the side wall portion 360 has a slit portion 361 continuous with the recess portion 353 and extending in the longitudinal direction of the inner member 320. The recess portion 353 has a trap portion 355 having the width W1 that narrows from the center position O1 of the upper end portion 350 toward the outer edge 351 of the upper end portion 350 in a plan view.

The hemostatic device 10 includes the check valve member 300 disposed in the inner cavity of the connector portion 210. In the natural state, the spring member 390 of the check valve member 300 forms the seal portion S inside the connector portion 210 by maintaining contact between the inner member 320 and the inner protrusion portion 315 formed in the outer member 310, and prevents the inner cavity 170a of the inflatable member 170 from being in fluid communication with the outside of the connector portion 210 via the connector portion 210. Furthermore, in the hemostatic device 10, the inner member 320 includes the main body 330 configured to be in contact with the inner protrusion portion 315 of the outer member 310, and the distal protrusion portion 340 that protrudes from the distal end of the main body 330 toward the distal opening 311 side. The distal protrusion portion 340 includes the upper end portion 350 and the side wall portion 360 connecting the upper end portion 350 and the main body 330. The upper end portion 350 has the recess portion 353 recessed toward the main body 330 side, and the recess portion 353 has the trap portion 355 having the width W1 that narrows from the center position O1 of the upper end portion 350 toward the outer edge 351 of the upper end portion 350 in a plan view. In the hemostatic device 10 described above, the foreign matter that has entered the inner cavity 316 of the outer member 310 through the distal opening 311 can be captured by the trap portion 355 included in the recess portion 353 located on the distal opening 311 side relative to the main body 330 of the inner member 320. Furthermore, in the hemostatic device 10, when the fluid is fed to the injection member 200 via the connector portion 210 in a state where the foreign matter has entered the connector portion 210, the foreign matter moved to the main body 330 side by the injected fluid can be captured by the trap portion 355. Therefore, the hemostatic device 10 can suppress movement of the foreign matter having entered the inner cavity 316 of the outer member 310 to the main body 330 side of the inner member 320. Thus, even in a case where the foreign matter enters the inner cavity 316 of the outer member 310, the hemostatic device 10 can prevent the foreign matter from being caught between the outer surface of the main body 330 of the inner member 320 and the inner protrusion portion 315 of the outer member 310 when the spring member 390 is in the natural state. Therefore, the hemostatic device 10 can prevent the sealing performance of the seal portion S from degrading due to the influence of the foreign matter that has entered the inner cavity 316 of the outer member 310. Thus, the hemostatic device 10 can prevent a decrease in the compressive force applied by the inflatable member 170 to the first puncture site p1 formed in the patient due to the influence of the foreign matter that has entered the inner cavity 316 of the outer member 310. Furthermore, in the hemostatic device 10, the side wall portion 360 included in the distal protrusion portion 340 has the slit portion 361 which is continuous with the recess portion 353 and extends in the longitudinal direction of the inner member 320. In the hemostatic device 10, when a pushing force in a direction away from the distal opening 311 is applied to the spring member 390 via the fluid injection device 900 inserted into the inner cavity 316 of the outer member 310 at the time of injecting the fluid into the inner cavity 170a of the inflatable member 170, the spring member 390 is compressed along the longitudinal direction of the connector portion 210. When the spring member 390 is compressed, the hemostatic device 10 moves the inner member 320 to the proximal side in accordance with the compression of the spring member 390. When the inner member 320 moves toward the proximal side in accordance with the compression of the spring member 390, the contact between the outer surface of the main body 330 of the inner member 320 and the inner protrusion portion 315 of the outer member 310 is released. In the hemostatic device 10, when the fluid is injected into the inner cavity 316 of the outer member 310 via the fluid injection device 900 in a state where the contact between the outer surface of the main body 330 of the inner member 320 and the inner protrusion portion 315 of the outer member 310 is released, the injected fluid can be smoothly moved from the distal protrusion portion 340 side toward the main body 330 side via the slit portion 361 formed in the side wall portion 360 connecting the upper end portion 350 and the main body 330. Therefore, when the inflatable member 170 is inflated using the fluid injection device 900, the hemostatic device 10 can smoothly feed the fluid into the inner cavity 170a of the inflatable member 170 via the connector portion 210 and the coupling portion 220.

Furthermore, in the hemostatic device 10, the recess portion 353 is connected to the slit portion 361 via the trap portion 355 (see Fig. 13).

In the hemostatic device 10 configured as described above, even when the fluid is injected into the inner cavity 316 of the outer member 310 in a state where the foreign matter exists in the recess portion 353 and the foreign matter moves toward the main body 330 by the injected fluid, the foreign matter is captured by the trap portion 355 connected to the slit portion 361 before reaching the slit portion 361. Therefore, the hemostatic device 10 can more reliably prevent the foreign matter from moving to the inner protrusion portion 315 of the main body 330 even in a case where the foreign matter enters the inner cavity of the connector portion 210.

Furthermore, in the hemostatic device 10, the upper end portion 350 has the first projection 371 formed on the outer edge 351 of the upper end portion 350 (see Figs. 13 and 14).

In the hemostatic device 10, since the first projection 371 formed at the outer edge 351 of the upper end portion 350 extends from the upper end portion 350 toward the distal side, in a case where the foreign matter enters the upper end portion 350 of the outer member 310 or in a case where the fluid is injected in a state where the foreign matter enters the upper end portion 350 of the outer member 310, it is possible to prevent the foreign matter from passing through the outer edge 351 of the upper end portion 350 and moving toward the main body 330 side.

Furthermore, in the hemostatic device 10, the upper end portion 350 has a plurality of upper surface portions 375 located on the same plane (see Figs. 13 and 15).

In the hemostatic device 10 configured as described above, when the distal tubular portion 910 of the fluid injection device 900 is inserted into the inner cavity of the connector portion 210 in order to inject the fluid into the inflatable member 170, the distal surface of the distal tubular portion 910 can be brought into contact with the plurality of upper surface portions 375 included in the upper end portion 350 over a wider area. The operator can firmly apply the pushing force to the inner member 320 by performing an operation of pushing the fluid injection device 900 in a state where the distal surface of the distal tubular portion 910 is in contact with the plurality of upper surface portions 375 over a wider area. Thus, the operator can easily move the inner member 320 to the proximal side. Therefore, the operator can bring the inner cavity of the connector portion 210 into an unsealed state by simple work of inserting the distal tubular portion 910 of the fluid injection device 900 into the inner cavity of the connector portion 210.

Furthermore, in the hemostatic device 10, the outer diameter D1 of the upper end portion 350 is greater than the inner diameter D2 of the distal opening 311 (see Fig. 9).

In the hemostatic device 10 configured as described above, the projection plane of the upper end portion 350 projected from the outer member 310 is larger than the projection plane of the distal opening 311 of the outer member 310 in a plan view as viewed from the distal side of the connector portion 210 as illustrated in Fig. 17. Therefore, in a case where the outer member 310 is viewed from the distal side of the connector portion 210 when the spring member 390 is in the natural state, the hemostatic device 10 can prevent an excessive gap (space that allows passage of the foreign matter) from being formed between the inner peripheral portion (inner wall portion) of the outer member 310 and the outer peripheral portion of the upper end portion 350. Therefore, the hemostatic device 10 can suitably prevent the foreign matter from passing through the gap between the inner peripheral portion of the outer member 310 and the outer peripheral portion of the upper end portion 350 and reaching the main body 330 even in a case where the foreign matter enters the inner cavity 316 of the outer member 310.

Furthermore, in the hemostatic device 10, the spring member 390 holds the end (the other end) 361b of the slit portion 361 located on the main body 330 side at a position on the distal opening 311 side relative to the inner protrusion portion 315 in the natural state, and the spring member 390 is configured to move the other end 361b of the slit portion 361 to a position on the proximal opening 312 side relative to the inner protrusion portion 315 when a pushing force in a direction toward the proximal opening 312 is applied to the spring member 390 (see Figs. 9, 10, and 11).

In the hemostatic device 10 configured as described above, when the spring member 390 is in the natural state, the other end 361b of the slit portion 361 located on the main body 330 side is held at the position on the distal opening 311 side relative to the inner protrusion portion 315, and thus it is possible to more reliably prevent the foreign matter from moving to the main body 330 via the slit portion 361 in the state where the seal portion S is formed. Furthermore, in the hemostatic device 10 configured as described above, when the pushing force in the direction toward the proximal opening 312 is applied to the spring member 390 and the hemostatic device is in the unsealed state, the other end 361b of the slit portion 361 is located at a position on the proximal opening 312 side relative to the inner protrusion portion 315. Therefore, the hemostatic device 10 can more reliably move the fluid injected from the distal side of the outer member 310 from one end 361a side located on the distal side relative to the inner protrusion portion 315 of the slit portion 361 toward the other end 361b side located on the proximal side relative to the inner protrusion portion 315. Thus, the hemostatic device 10 can smoothly feed the fluid into the inner cavity 170a of the inflatable member 170 in the unsealed state.

Furthermore, in the hemostatic device 10, the recess portion 353 has a cutout portion 355a connecting the center position O1 of the upper end portion 350 and the side surface of the upper end portion 350. The width W2 of the cutout portion 355a gradually decreases toward the main body 330 side (see Fig. 14).

In the hemostatic device 10 configured as described above, when the foreign matter that has reached the recess portion 353 moves toward the main body 330 side, the foreign matter can be captured by the cutout portion 355a before the foreign matter reaches the slit portion 361. Furthermore, since the width W2 of the cutout portion 355a gradually decreases toward the main body 330 side, when the foreign matter that has reached the recess portion 353 moves from the distal side of the upper end portion 350 toward the main body 330 side, the foreign matter can be more reliably captured by the portion where the width W2 of the cutout portion 355a is formed to be smaller. Therefore, the hemostatic device 10 can more reliably prevent the foreign matter from moving to the main body 330 even in a case where the foreign matter enters the inner cavity of the connector portion 210.

Furthermore, in the hemostatic device 10, the cutout portions 355a are disposed at four locations at equal intervals in the circumferential direction of the upper end portion 350 with reference to the center position O1 of the upper end portion 350 (see Fig. 15).

The hemostatic device 10 configured as described above can move the fluid from the upper end portion 350 side toward the side wall portion 360 side via the four cutout portions 355a when the distal tubular portion 910 of the fluid injection device 900 is brought into contact with the distal protrusion portion 340 to perform the operation of injecting the fluid. Furthermore, since the cutout portions 355a are disposed at equal intervals in the circumferential direction of the upper end portion 350, the fluid can be radially moved from the center position O1 side of the upper end portion 350 toward each of the four directions in which the cutout portions 355a extend. Therefore, the hemostatic device 10 can smoothly feed the fluid into the inner cavity 170a of the inflatable member 170. Furthermore, in the hemostatic device 10, since the cutout portions 355a are provided at four locations in the upper end portion 350 at equal intervals in the circumferential direction of the recess portion 353, it is possible to prevent the rigidity of the upper end portion 350 from excessively decreasing due to the provision of the cutout portions 355a while smoothly moving the fluid as described above.

Furthermore, in the hemostatic device 10, the slit portion 361 has a predetermined depth L1 along a direction from the side wall portion 360 side toward the center position O1 side of the upper end portion 350 (see Fig. 13).

In the hemostatic device 10 configured as described above, since the slit portion 361 has the predetermined depth L1 along the direction from the side wall portion 360 side toward the center position O1 side of the upper end portion 350, even in a case where the foreign matter passes through the trap portion 355, the foreign matter can be captured by the slit portion 361 connected to the trap portion 355. Therefore, the hemostatic device 10 can more reliably prevent the foreign matter that enters the inner cavity of the connector portion 210 from moving to the main body 330.

Furthermore, in the hemostatic device 10, the slit portion 361 extends with a substantially constant width W3 along the longitudinal direction of the side wall portion 360 (see Fig. 16).

In the hemostatic device 10 configured as described above, when the fluid is injected from the distal protrusion portion 340 side at the time of injecting the fluid into the inner cavity 170a of the inflatable member 170, the fluid is smoothly guided toward the main body 330 side along the slit portion 361 having the substantially constant width W3. Therefore, when the operator operates the fluid injection device 900 to inject the fluid into the inner cavity 170a of the inflatable member 170, the hemostatic device 10 can effectively reduce the resistance of the hand felt by the operator.

Furthermore, the hemostatic device 10 includes the first region 381 in which the upper end portion 350 includes the upper surface portion 375 and the second region 382 forming the bottom portion 353a of the recess portion 353, and the end (the other end 361b) located on the main body 330 side of the slit portion 361 is located on the main body 330 side relative to the second region 382 (see Fig. 14).

In the hemostatic device 10 configured as described above, since the other end 361b of the slit portion 361 located on the main body 330 side is located on the main body 330 side relative to the second region 382, when the fluid is injected from the distal protrusion portion 340 side at the time of injecting the fluid into the inner cavity 170a of the inflatable member 170, the fluid smoothly moves to the main body 330 side via the other end 361b of the slit portion 361. Therefore, when the operator operates the fluid injection device 900 to inject the fluid into the inner cavity 170a of the inflatable member 170, the hemostatic device 10 can effectively reduce the resistance of the hand felt by the operator.

Furthermore, in the present embodiment, it is possible to provide a hemostatic device assembly including the hemostatic device 10 and the fluid injection device 900 including the distal tubular portion 910 that abuts the upper end portion 350. In such a hemostatic device assembly, the upper surface portion 375 that the distal tubular portion 910 abuts in the upper end portion 350 has the outer diameter D3 greater than that of the distal tubular portion 910 in a plan view (see Figs. 15 and 18).

In the hemostatic device assembly configured as described above, when the distal tubular portion 910 is brought into contact with the upper surface portion 375 of the upper end portion 350, the upper surface portion 375 and the distal tubular portion 910 can be brought into contact with each other over a wider area while preventing the distal tubular portion 910 from protruding outward from the outer edge of the upper surface portion 375. Therefore, the hemostatic device 10 can more firmly apply a pushing force to the inner member 320 when the distal tubular portion 910 of the fluid injection device 900 is inserted into the inner cavity of the connector portion 210 and the inner member 320 is pushed. Thus, the hemostatic device 10 can more smoothly feed the fluid into the inner cavity 170a of the inflatable member 170 when the inflatable member 170 is inflated.

Next, modification examples of the above-described embodiment will be described. In the description of the modification examples, the descriptions of the members, the use process of the hemostatic device, and the like already described will be omitted, as appropriate. Furthermore, contents that are not particularly described in the modification examples can be the same as those in the above-described embodiment.

### <First Modification Example>

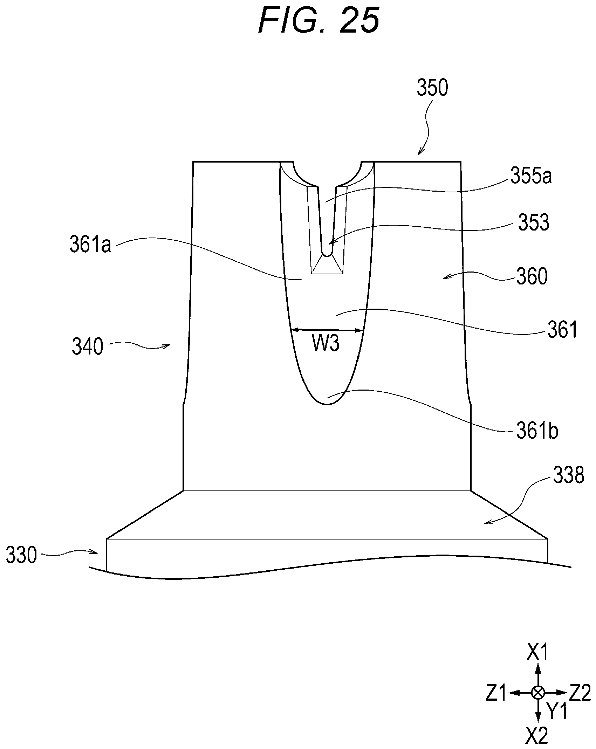

Fig. 25 illustrates an inner member according to the first modification example. Fig. 25 is a view corresponding to Fig. 16 of the embodiment described above.

As illustrated in Fig. 25, for example, the slit portion 361 can be configured such that the width W3 gradually decreases toward the main body 330 side.

### <Second Modification Example>

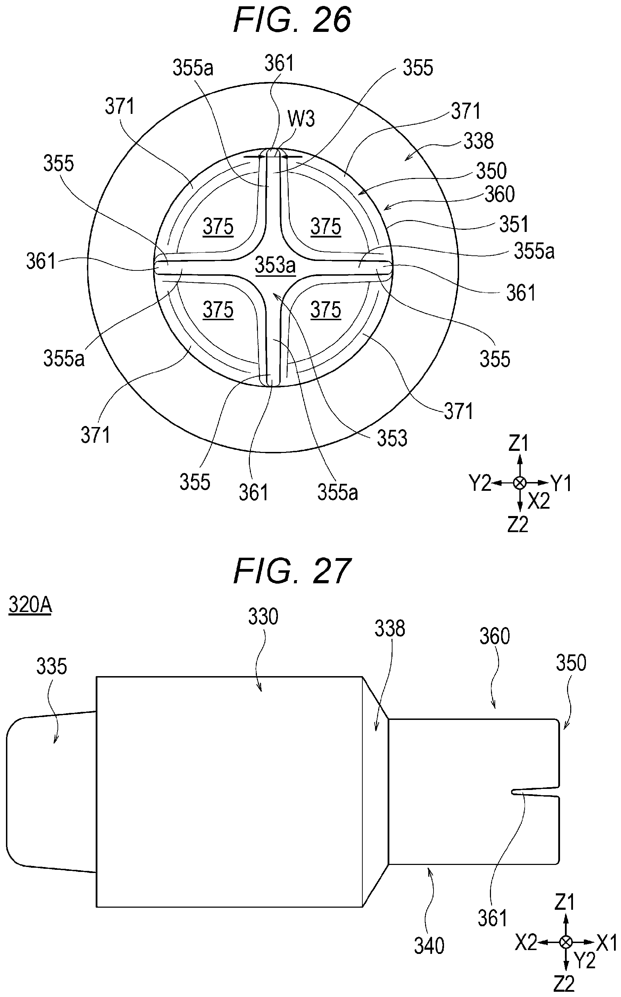

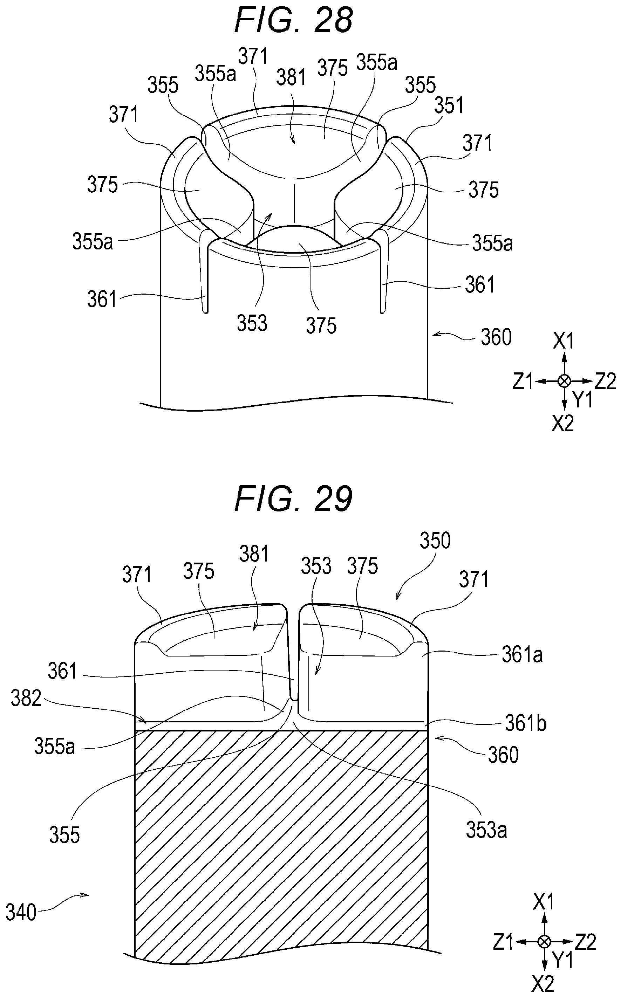

Figs. 26 to 29 illustrate an inner member 320A according to a second modification example. Fig. 26 is a plan view of the inner member 320A according to the second modification example as viewed from the distal side, Fig. 27 is a side view of the inner member 320A according to the second modification example, Fig. 28 is a perspective view illustrating the upper end portion 350 and the side wall portion 360 of the inner member 320A according to the second modification example, and Fig. 29 is a partial cross-sectional view of the upper end portion 350 and the side wall portion 360 of the inner member 320A according to the second modification example.

As illustrated in Fig. 29, in the inner member 320A according to the second modification example, the other end 361b of the slit portion 361 located on the main body 330 side is located at substantially the same position in the height direction of the distal protrusion portion 340 as that of the second region 382 where the bottom portion 353a of the recess portion 353 is formed.

In the hemostatic device according to the second modification example, there is no portion where the width W3 of each slit portion 361 increases in the plan view illustrated in Fig. 26, and the minimum portion of the width W1 of the trap portion 355 and the width W3 of the slit portion 361 are substantially constant (in the hemostatic device 10 of the above-described embodiment, the width W3 of each slit portion 361 increases so as to be greater than the width W1 of the trap portion 355 in the plan view illustrated in Fig. 15), and thus the foreign matter is easily captured by each slit portion 361. However, in the hemostatic device according to the second modification example, the width W3 of the slit portion 361 is the same as the width W2 (minimum width) of the cutout portion 355a (in the hemostatic device 10 of the above-described embodiment, the width W3 of the slit portion 361 > the width W2 of the cutout portion 355a is satisfied), and the gap (space) between the inner peripheral portion of the outer member 310 and the outer peripheral portion of the inner member 320 becomes narrow, and thus the injection resistance of the fluid increases. Therefore, in a case where the injection resistance of the fluid is to be reduced, the hemostatic device preferably adopts the structure of the hemostatic device 10 according to the above-described embodiment.

### <Third Modification Example>

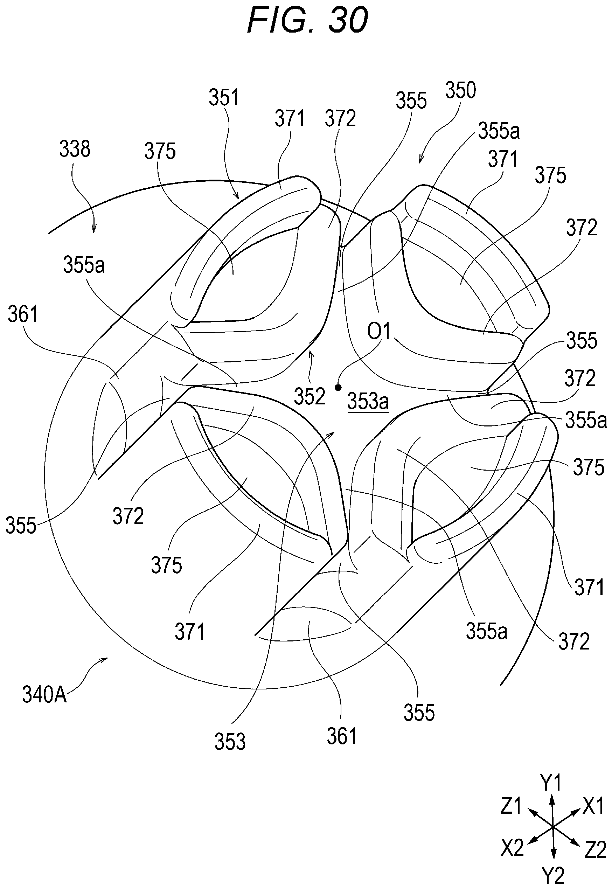

Fig. 30 is a perspective view of a distal protrusion portion 340A of an inner member according to a third modification example.

The upper end portion 350 of the distal protrusion portion 340A of the inner member according to the third modification example has a second projection 372 formed at the inner edge 352 of the upper end portion 350.

The above-described "inner edge 352 of the upper end portion 350" means a portion of the upper end portion 350 closest to the center position O1 of the upper surface portion 375. As described in the present modification example, in a case where a plurality of upper surface portions 375 are formed in the upper end portion 350, the inner edge 352 of the upper surface portion 375 can be defined by an edge located on the center position O1 side of the upper end portion 350 in each upper surface portion 375.

The inner member according to the third modification example includes a first projection 371 formed at the outer edge 351 of the upper end portion 350 and a second projection 372 formed at the inner edge 352 of the upper end portion 350. The upper surface portion 375 having a flat surface shape has a recess shape surrounded by the first projection 371 and the second projection 372.

The inner member according to the third modification example can more reliably capture the foreign matter having entered the inner cavity of the connector portion 210 by the upper surface portion 375 formed in the recess shape as described above. However, in the inner member according to the third modification example, when the fluid injection device 900 is pushed against the upper end portion 350, the distal tubular portion 910 of the fluid injection device 900 cannot be brought into contact with the upper surface portion 375, and the range in which the distal tubular portion 910 comes into contact is limited only to the upper end of the second projection 372. Therefore, there is a possibility that the operator cannot sufficiently transmit the pushing force to the inner member via the distal tubular portion 910 of the fluid injection device 900. Therefore, in the case of enabling the sufficient pushing force to be transmitted to the inner member as described above, it is preferable to adopt a configuration in which the second projection 372 is not provided at the inner edge 352 of the upper end portion 350 as described in the above-described embodiment (see Fig. 13).

### <Fourth Modification Example>

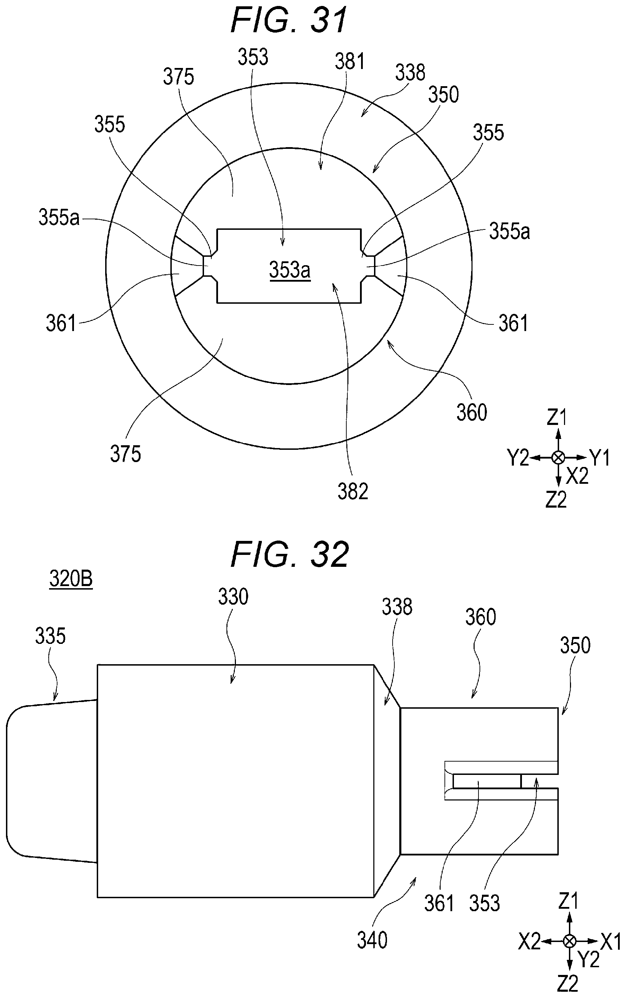

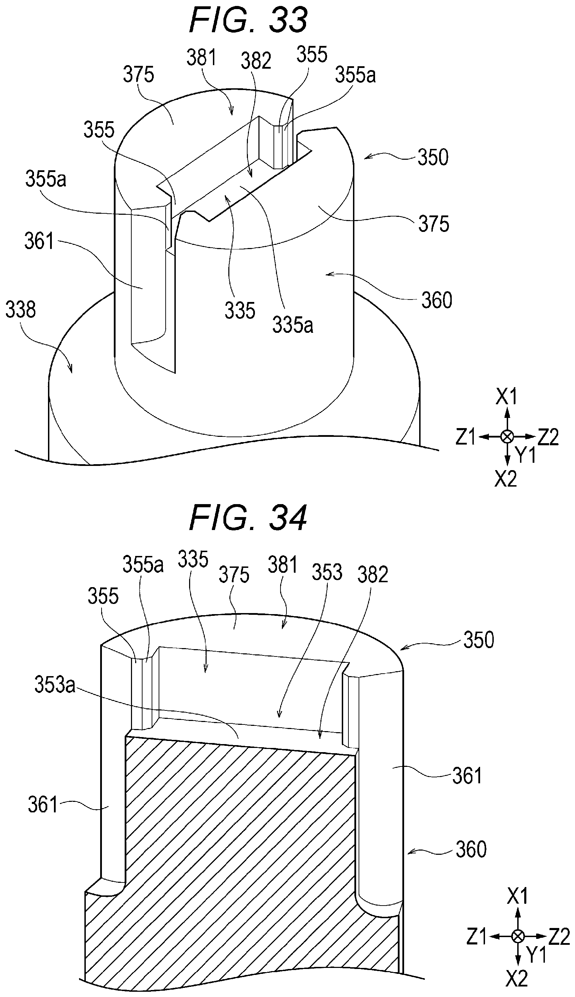

Figs. 31 to 34 illustrate an inner member 320B according to a fourth modification example. Fig. 31 is a plan view of the inner member 320B according to the fourth modification example as viewed from the distal side, Fig. 32 is a side view of the inner member 320B according to the fourth modification example, Fig. 33 is a perspective view illustrating the upper end portion 350 and the side wall portion 360 of the inner member 320B according to the fourth modification example, and Fig. 34 is a partial cross-sectional view of the upper end portion 350 and the side wall portion 360 of the inner member 320B according to the fourth modification example.

The inner member 320B according to the fourth modification example is different from the inner member 320 of the above-described embodiment in the shape of the recess portion 353 in a plan view.

The recess portion 353 of the inner member 320B has a substantially rectangular shape having long sides along the lateral direction (directions of arrows Y1 and Y2) in a plan view illustrated in Fig. 31. Two upper surface portions 375 are formed so as to sandwich the recess portion 353 therebetween.

As illustrated in Figs. 31 and 34, the trap portion 355 is formed in a portion extending from the vicinity of both ends of the recess portion 353 in the longitudinal direction toward the slit portion 361 side. Furthermore, the cutout portion 355a of the recess portion 353 is located between the trap portion 355 and the slit portion 361 in the plan view illustrated in Fig. 31. The slit portion 361 is provided at a position corresponding to the position where the trap portion 355 and the cutout portion 355a are formed. Two slit portions 361 are formed so as to sandwich the recess portion 353 therebetween.

When the operator injects the fluid into the inner cavity 170a of the inflatable member 170 using the fluid injection device 900, the distal tubular portion 910 of the fluid injection device 900 is brought into contact with two upper surface portions 375. When the operator operates the fluid injection device 900 in a state where the distal tubular portion 910 of the fluid injection device 900 is in contact with the upper surface portion 375 of the inner member 320B, a larger amount of fluid can be fed through the recess portion 353 extending in a relatively wide range in the lateral direction including the center position O1 of the upper end portion 350, and thus the fluid can be more smoothly moved toward the main body 330 side. However, since the recess portion 353 of the inner member 320B according to the fourth modification example forms a space extending in a relatively wide range in the lateral direction of the upper end portion 350, the effect of capturing the foreign matter is less likely to be exhibited as compared with the inner member 320 (see Fig. 15) including the recess portion 353 formed in the cross-shaped cutout described in the above-described embodiment. Therefore, in a case where the hemostatic device is configured to more sufficiently exhibit the effect of capturing the foreign matter, it is preferable to adopt the same configuration as that of the inner member 320 of the above-described embodiment.

### <Fifth Modification Example>

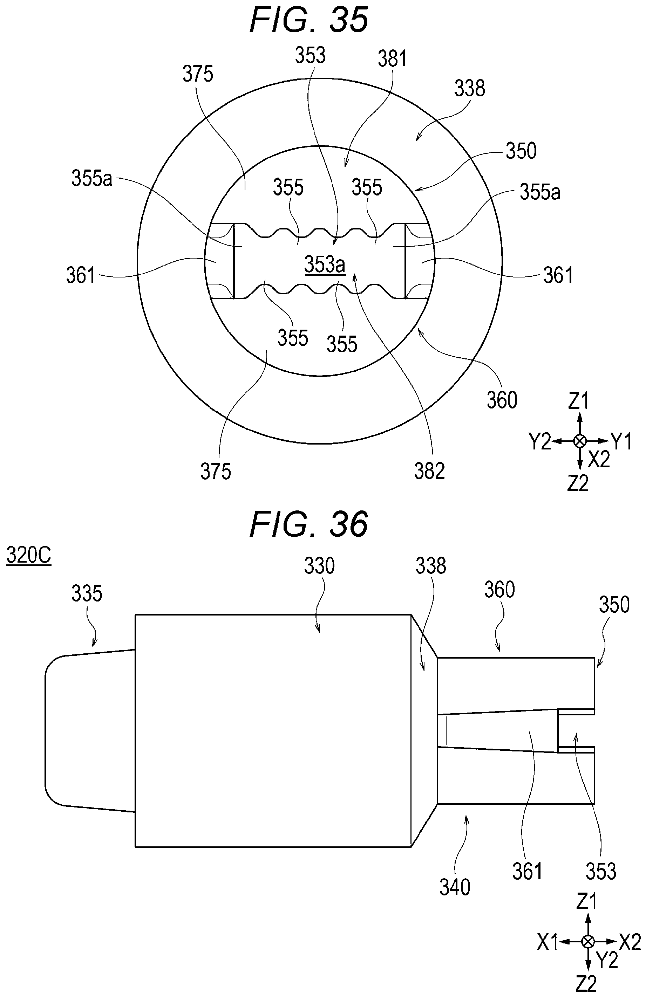

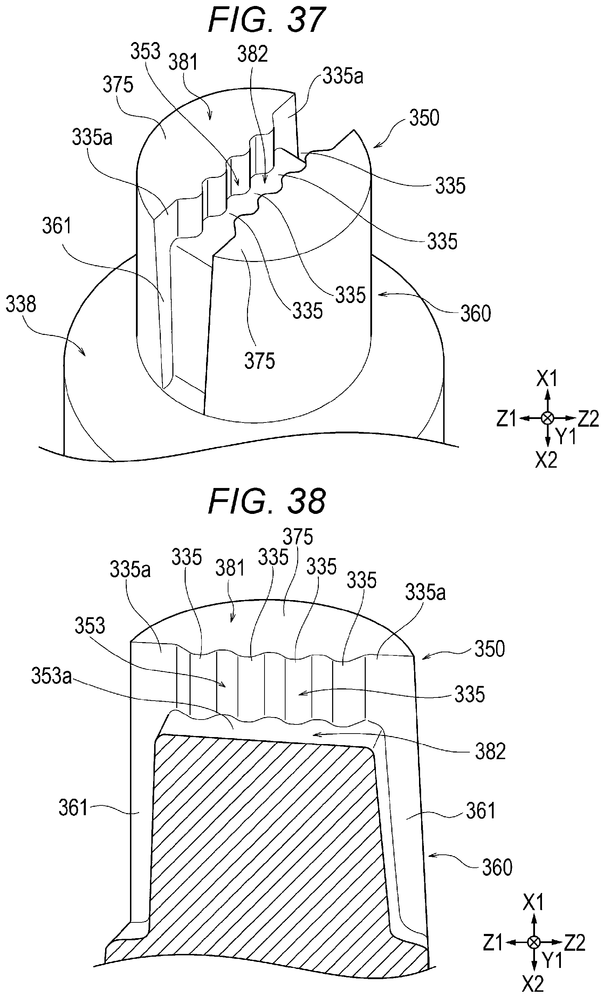

Figs. 35 to 38 illustrate an inner member 320C according to a fifth modification example. Fig. 35 is a plan view of the inner member 320C according to the fifth modification example as viewed from the distal side, Fig. 36 is a side view of the inner member 320C according to the fifth modification example, Fig. 37 is a perspective view illustrating the upper end portion 350 and the side wall portion 360 of the inner member 320C according to the fifth modification example, and Fig. 38 is a partial cross-sectional view of the upper end portion 350 and the side wall portion 360 of the inner member 320C according to the fifth modification example.

Similarly to the inner member 320B described in the fourth modification example, the inner member 320C according to the fifth modification example is different from the inner member 320 of the above-described embodiment in the shape of the recess portion 353 in a plan view.

The recess portion 353 of the inner member 320C extends by a predetermined length along the lateral direction (directions of arrows Y1 and Y2) in the plan view illustrated in Fig. 35. Two upper surface portions 375 are formed so as to sandwich the recess portion 353 therebetween.

As illustrated in Figs. 35 and 37, a plurality of trap portions 355 are formed at predetermined intervals along the direction in which the recess portion 353 extends. Therefore, the recess portion 353 has a shape extending in a wave shape in the plan view illustrated in Fig. 35. The cutout portion 355a is located near both ends of the recess portion 353 in the lateral direction in the plan view illustrated in Fig. 35. The slit portion 361 is provided at a position corresponding to the position where the cutout portion 355a is formed. Two slit portions 361 are formed so as to sandwich the recess portion 353 therebetween.

When the operator injects the fluid into the inner cavity 170a of the inflatable member 170 using the fluid injection device 900, the distal tubular portion 910 of the fluid injection device 900 is brought into contact with two upper surface portions 375. When the operator operates the fluid injection device 900 in a state where the distal tubular portion 910 of the fluid injection device 900 is in contact with the upper surface portion 375 of the inner member 320C, a larger amount of fluid can be fed through the recess portion 353 extending in a relatively wide range in the lateral direction including the center position O1 of the upper end portion 350, and thus the fluid can be more smoothly moved toward the main body 330 side. However, since the recess portion 353 of the inner member 320C according to the fifth modification example forms a space extending in a relatively wide range in the lateral direction of the upper end portion 350 similarly to the recess portion of the inner member 320B according to the fourth modification example, the effect of capturing the foreign matter is less likely to be exhibited as compared with the inner member 320 (see Fig. 15) including the recess portion 353 formed in the cross-shaped cutout described in the above-described embodiment. Therefore, in a case where the hemostatic device is configured to more sufficiently exhibit the effect of capturing the foreign matter, it is preferable to adopt the same configuration as that of the inner member 320 of the above-described embodiment.

While the hemostatic device according to the present invention has been described above using the embodiment and the modification examples, the present invention is not limited only to the content described in the specification and can be appropriately changed based on the description of the claims.

The shape, size, and the like of each part of the hemostatic device are not particularly limited as long as a puncture site can be compressed to perform hemostasis using the inflatable member disposed at the puncture site, and can be appropriately changed.

The specific shapes of the inflatable member and the support member are not limited to the shapes illustrated in the embodiment.

The hemostatic device can also be configured to perform hemostasis from a site other than the puncture site formed in the hand. For example, the hemostatic device can also be applied to hemostasis at a puncture site formed on an arm, a leg, or the like, of a patient using the inflatable member of the embodiment.

The present application is based on Japanese Patent Application No. 2023-170329 filed on September 29, 2023, the disclosure content of which is incorporated herein by reference in its entirety.

### Reference Signs List

10 Hemostatic device
100 Cover member
120 Sheet material
170 Inflatable member
170a Inner cavity of inflatable member
200 Injection member
210 Connector portion
220 Coupling portion
300 Check valve member
310 Outer member
311 Distal opening
312 Proximal opening
313 Insertion port
315 Inner protrusion portion
316 Inner cavity of outer member
320 Inner member
320A Inner member
320B Inner member
320C Inner member
330 Main body
338 Seal-forming portion
340 Distal protrusion portion
340A Distal protrusion portion
350 Upper end portion
351 Outer edge
352 Inner edge
353 Recess portion
353a Bottom portion
355 Trap portion
355a Cutout portion
360 Side wall portion
361 Slit portion
361a One end of slit portion
361b The other end (end) of slit portion
371 First projection
372 Second projection
375 Upper surface portion
381 First region
382 Second region
390 Spring member
400 Connection member
410a Inner cavity of connection member
500 Support member
610 First band body
620 Second band body
630 Third band body
800 Introducer
900 Fluid injection device
910 Distal tubular portion
912 Injection port
O1 Center position of upper end portion
S Seal portion
D1 Outer diameter of upper end portion
D2 Inner diameter of distal opening
D3 Outer diameter of upper surface portion
D4 Outer diameter of distal tubular portion
D5 Inner diameter of injection port
D6 Outer diameter of inner peripheral portion of recess portion
L1 Depth of slit portion
W1 Width of trap portion
W2 Width of cutout portion
W3 Width of slit portion
H Hand
H1 Right hand
Ar Forearm
fb Interdigital portion
Sb Snuff box
B1 Metacarpal bone of index finger
B2 Metacarpal bone of thumb
T1 Extensor pollicis longus muscle tendon
T2 Extensor pollicis brevis muscle tendon
V1 Blood vessel (distal radial artery)
V2 Artery located at snuff box
p1 First puncture site (puncture site)
p2 Second puncture site (puncture site)

## Claims

1. A hemostatic device comprising:
a cover member configured to cover a puncture site formed on a patient;
an inflatable member connected to the cover member and configured to compress the puncture site; and
an injection member connected to the inflatable member and configured to be capable of injecting a fluid into the inflatable member,
wherein the injection member includes a connector portion, and a coupling portion that couples an inner cavity of the connector portion and an inner cavity of the inflatable member,
the connector portion includes a check valve member configured to be capable of controlling communication between the inner cavity of the connector portion and an outside, and a connection member that connects the check valve member and the coupling portion,
the check valve member includes an outer member, an inner member located in an inner cavity of the outer member, and a spring member connected to the inner member,
the outer member includes a distal opening configured to expose a part of the inner member to the outside, a proximal opening communicating between the inner cavity of the outer member and an inner cavity of the connection member, and an inner protrusion portion configured to prevent movement of the inner member toward the distal opening side,
the spring member is located between the proximal opening and the inner protrusion portion so as to maintain contact between an outer surface of the inner member and the inner protrusion portion in a natural state,
the inner member includes a main body configured to contact the inner protrusion portion in the natural state, and a distal protrusion portion protruding from a distal end of the main body toward the distal opening side,
the distal protrusion portion includes an upper end portion and a side wall portion connecting the upper end portion and the main body,
the upper end portion includes a recess portion recessed toward the main body side,
the side wall portion includes a slit portion continuous with the recess portion and extending in a longitudinal direction of the inner member, and
the recess portion includes a trap portion having a width that narrows from a center position of the upper end portion toward an outer edge of the upper end portion in a plan view.

2. The hemostatic device according to claim 1, wherein the recess portion is connected to the slit portion via the trap portion.

3. The hemostatic device according to claim 1, wherein the upper end portion includes a first projection formed at the outer edge of the upper end portion.

4. The hemostatic device according to claim 3, wherein the upper end portion includes a plurality of upper surface portions located on a same plane.

5. The hemostatic device according to claim 4, wherein the upper surface portion includes a second projection formed at an inner edge of the upper surface portion.

6. The hemostatic device according to claim 1, wherein an outer diameter of the upper end portion is greater than an inner diameter of the distal opening.

7. The hemostatic device according to claim 1, wherein the spring member holds an end of the slit portion located on the main body side at a position on the distal opening side relative to the inner protrusion portion in the natural state, and
the spring member moves the end of the slit portion to a position on the proximal opening side relative to the inner protrusion portion when a pushing force in a direction toward the proximal opening is applied to the spring member.

8. The hemostatic device according to claim 1, wherein the recess portion includes a cutout portion connecting the center position of the upper end portion and a side surface of the upper end portion, and
the cutout portion has a width that gradually narrows toward the main body side.

9. The hemostatic device according to claim 8, wherein the cutout portions are disposed at four locations at equal intervals in a circumferential direction of the upper end portion with reference to the center position of the upper end portion.

10. The hemostatic device according to claim 1, wherein the slit portion has a predetermined depth along a direction from the side wall portion side toward the center position side of the upper end portion.

11. The hemostatic device according to claim 1, wherein the slit portion extends with a substantially constant width along a longitudinal direction of the side wall portion.

12. The hemostatic device according to claim 1, wherein the upper end portion includes a first region including the upper surface portion and a second region forming a bottom portion of the recess portion, and
an end of the slit portion located on the main body side is located on the main body side relative to the second region.

13. A hemostatic device set comprising:
the hemostatic device according to any one of claims 1 to 12; and
a fluid injection device including a distal tubular portion that abuts the upper end portion,
wherein an upper surface portion that the distal tubular portion abuts in the upper end portion has an outer diameter greater than that of the distal tubular portion in a plan view.
